(19) Europäisches Patentamt
European Patent Office
Office européen des brevets

(11) EP 3 983 783 B1

(12) # FASCICULE DE BREVET EUROPEEN

(45) Date de publication et mention de la délivrance du brevet:
**05.07.2023 Bulletin 2023/27**

(21) Numéro de dépôt: **20750325.1**

(22) Date de dépôt: **08.07.2020**

(51) Classification Internationale des Brevets (IPC):
***G01N 21/69*** (2006.01) ***G01N 21/76*** (2006.01)
***G01N 33/48*** (2006.01) ***G01N 1/40*** (2006.01)

(52) Classification Coopérative des Brevets (CPC):
**G01N 21/76; G01N 1/4044**

(86) Numéro de dépôt international:
**PCT/FR2020/051209**

(87) Numéro de publication internationale:
**WO 2021/009438 (21.01.2021 Gazette 2021/03)**

(54) **PROCÉDÉ DE DÉTECTION DU SCATOL DANS UN ÉCHANTILLON D'UN TISSU ADIPEUX DE PORC**

VERFAHREN ZUR DETEKTION VON SKATOL IN EINER PROBE VON SCHWEINEFETTGEWEBE

METHOD FOR DETECTING SKATOLE IN A SAMPLE OF PIG ADIPOSE TISSUE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.07.2019 FR 1907882**

(43) Date de publication de la demande:
**20.04.2022 Bulletin 2022/16**

(73) Titulaire: **Commissariat à l'Energie Atomique et aux Energies Alternatives**
**75015 Paris (FR)**

(72) Inventeurs:
- **SCORSONE, Emmanuel**
**78114 MAGNY-LES-HAMEAUX (FR)**
- **HAMEL, Matthieu**
**50100 CHERBOURG (FR)**

(74) Mandataire: **Brevalex**
**Tour Trinity**
**1 Bis Esplanade de La Défense**
**CS 60347**
**92035 Paris La Défense Cedex (FR)**

(56) Documents cités:
- **TAKEYOSHI OKAJIMA ET AL: "Chemiluminescence of 3-methylindole based on electrogeneration of superoxide ion in acetonitrile solutions", JOURNAL OF ELECTROANALYTICAL CHEMISTRY, vol. 523, no. 1-2, avril 2002 (2002-04), pages 34-39, XP055675127, AMSTERDAM, NL ISSN: 1572-6657, DOI: 10.1016/S0022-0728(02)00684-8 cité dans la demande**
- **D.K.BELGHITI ET AL.: "Simultaneous detection of indole and 3-methylindole using boron-doped diamond electrodes", PHYS.STATUS SOLIDI A, vol. 213, no. 10, 23 septembre 2016 (2016-09-23), pages 2662-2671, XP009519302, DOI: 10.1002/pssa.201600187**
- **KATERYNA MUZYKA ET AL: "Boron-doped diamond: current progress and challenges in view of electroanalytical applications", ANALYTICAL METHODS, vol. 11, no. 4, janvier 2019 (2019-01), pages 397-414, XP055674950, GBR ISSN: 1759-9660, DOI: 10.1039/C8AY02197J**
- **JOHN W. PENSABENE ET AL: "Indole and Skatole in Fresh Pork as Possible Markers of Fecal Contamination+", JOURNAL OF FOOD PROTECTION, vol. 59, no. 6, juin 1996 (1996-06), pages 663-665, XP055674929, US ISSN: 0362-028X, DOI: 10.4315/0362-028X-59.6.663 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 983 783 B1

- LIU XIAOYE ET AL: "Feasibility of boar taint classification using a portable Raman device", MEAT SCIENCE, vol. 116, 6 février 2016 (2016-02-06), pages 133-139, XP029456703, ISSN: 0309-1740, DOI: 10.1016/J.MEATSCI.2016.02.015
- VERPLANKEN KAAT ET AL: "Rapid method for the simultaneous detection of boar taint compounds by means of solid phase microextraction coupled to gas chromatography/mass spectrometry", JOURNAL OF CHROMATOGRAPHY A, ELSEVIER, AMSTERDAM, NL, vol. 1462, 28 juillet 2016 (2016-07-28), pages 124-133, XP029688892, ISSN: 0021-9673, DOI: 10.1016/J.CHROMA.2016.07.077 cité dans la demande

## Description

### Domaine technique

L'invention relève du domaine de l'agroalimentaire et, en particulier, du domaine de la production et de la distribution de la viande de porc.

Plus spécifiquement, l'invention se rapporte à un procédé permettant de détecter la présence de scatol, ou 3-méthylindole (3-MIH), dans un échantillon d'un tissu adipeux de porc et, si celui-ci est présent, d'en déterminer la teneur et ce, avec une très grande sensibilité et une très grande spécificité vis-à-vis des autres composés susceptibles d'être également présents dans le tissu adipeux de porc.

Le scatol étant, avec l'androstérone, responsable d'une odeur forte et désagréable, dite odeur de verrat, qui est dégagée pendant la cuisson de la viande de porcs mâles entiers, l'invention est, en premier lieu, susceptible d'être mise en oeuvre pour trier dans les abattoirs les carcasses de porcs mâles entiers en vue d'isoler celles dont la viande est porteuse d'odeur de verrat et de les orienter vers un circuit de transformation adapté.

Elle est également susceptible d'être mise en oeuvre comme outil de recherche, notamment pour étudier les facteurs influençant la production de scatol dans les tissus adipeux des porcs mâles entiers en vue de développer des méthodes permettant de prévenir ou de réduire l'odeur de verrat, par exemple par sélection génétique ou par modification des conditions d'élevage (alimentation, conditions de stabulation, composition des groupes d'animaux en termes d'âge, de sexe, etc).

### État de la technique antérieure

L'odeur de verrat découle principalement d'une accumulation du scatol et de l'androstérone et, dans une moindre mesure, de l'indole dans les tissus adipeux - ou tissus gras - des porcs mâles entiers, c'est-à-dire non castrés.

Cette odeur, qui se dégage pendant la cuisson de la viande de porc, est généralement considérée comme nauséabonde par les consommateurs.

Historiquement, pour prévenir l'odeur de verrat, les porcs mâles étaient castrés à un âge antérieur à leur maturité sexuelle.

Depuis une dizaine d'années toutefois, des raisons éthiques, de bien-être animal mais également économiques amènent un certain nombre d'éleveurs à ne plus castrer les porcs mâles avec l'appui de l'Union Européenne.

De ce fait, il est nécessaire de disposer d'une méthode permettant de détecter de manière fiable, sur les chaînes d'abattage, les carcasses de porcs mâles entiers dont la viande est porteuse d'odeur de verrat.

Le tri des carcasses à partir d'une détection du scatol en abattoir pose de nombreuses difficultés.

Les raisons de ces difficultés sont multiples.

En premier lieu, la détection du scatol dans les tissus gras des porcs nécessite une technique de détection extrêmement sensible. En effet, le seuil de rejet d'une viande de porc par le consommateur est fixé à environ 0,2 $\mu$g de scatol par gramme de tissu adipeux. En termes analytiques, cela signifie que, dans le cas par exemple d'une extraction complète du scatol présent dans un gramme de tissu adipeux et concentré dans un mL d'un solvant approprié, l'objectif revient à pouvoir détecter un seuil de concentration du scatol de 1,53 $\mu$mol/L dans ce mL de solution. De même, après chauffage du tissu adipeux et volatilisation des molécules de scatol, la concentration du scatol en phase vapeur est de l'ordre de quelques dizaines de ppb, ce qui ne rend pas plus facile la mesure du scatol dans une telle phase.

Une autre difficulté est celle de la sélectivité de la détection du scatol. En effet, le scatol étant un composé indolique qui est issu de la dégradation du tryptophane par les bactéries intestinales, il est présent dans le gras de porc avec d'autres composés indoliques comme l'indole, qui sont susceptibles d'interférer dans une détection du scatol en provoquant des faux positifs.

Enfin, s'ajoutent à cela :

- des contraintes de cadence, la détection du scatol devant pouvoir être réalisée sur plusieurs centaines de carcasses par jour pour la plupart des abattoirs ;
- des contraintes liées aux conditions qui règnent dans les abattoirs qui sont des milieux non contrôlés présentant de fortes variations de température au cours de l'année et un fort taux d'humidité ;
- des contraintes de coût, la détection du scatol ne devant pas impacter de façon significative le prix de vente ultérieur de la viande de porc ;
- une variabilité dans la composition des tissus adipeux prélevés sur les carcasses (teneur en eau, teneur en sang, etc) ; et
- un problème de traçabilité des échantillons analysés en abattoir.

Des procédés de détection du scatol sont connus.

Les procédés les plus répandus font appel à une chromatographie en phase liquide à haute performance ou à une chromatographie en phase gazeuse (cf., par exemple, K. Verplanken et al, Journal of Chromatography A 2016, 1462, 124-133). Ces procédés ne sont pas adaptés à une utilisation en abattoir car ils nécessitent des temps d'analyse longs, des opérateurs hautement qualifiés, un appareillage et des consommables onéreux et un niveau de maintenance élevé. De plus, compte-tenu de la complexité de la matrice graisseuse, ils présentent généralement un niveau de sélectivité insuffisant.

Récemment, le Danemark a annoncé disposer d'une méthode automatisée reposant sur la spectrométrie de masse après un traitement adéquat des échantillons. Mais cette méthode est également coûteuse et probablement peu adaptée aux conditions d'utilisation en abattoir. Elle souffre de plus d'un problème de traçabilité des échantillons qui sont analysés en série dans le spectromètre.

Des procédés immunologiques, notamment basés sur un dosage fluoro-immunologique utilisant l'europium(III) en tant que marqueur fluorescent, ont été étudiés (cf., par exemple, M. P. Aguilar-Caballos et al, Analytica Chimica Acta 2002, 460, 271-277) mais ces études restent marginales en raison la non disponibilité d'anticorps spécifiques.

Un procédé de détection colorimétrique du scatol utilisant le réactif d'Ehrlich a été proposé (cf. la demande internationale WO 83/00928). Ce procédé, qui est simple à mettre en oeuvre et peu coûteux, a été utilisé dans des abattoirs danois dans les années 1990. Malheureusement, il a dû être abandonné en raison du manque de sélectivité du réactif d'Ehrlich, en particulier vis-à-vis de l'indole, et partant de l'absence de fiabilité des résultats.

Des procédés électrochimiques ont également été proposés pour la détection du scatol (cf., par exemple, la demande de brevet européen 2 966 441). Ces procédés consistent à mesurer le courant d'oxydation du scatol à la surface d'une électrode, le plus souvent en carbone (diamant ou carbone vitreux). Ces procédés présentent une très grande sensibilité mais ils souffrent d'un problème majeur de sélectivité en raison, là également, de la complexité de la matrice graisseuse.

Plus récemment, des méthodes faisant appel à la spectroscopie Raman ont été proposées (cf., par exemple, X. Liu et al, Meat Science 2016, 116, 133-139). Ces méthodes ont une approche globale consistant à analyser simultanément l'androsténone et le scatol dans un tissu adipeux. Toutefois, les résultats présentés jusqu'à présent mettent en évidence un nombre de faux positifs et de faux négatifs inacceptable.

Enfin, des nez électroniques ont été développés (cf., par exemple, J. S. Vestergaard et al, Meat Science 2006, 74, 564-577). Le fonctionnement de ces nez électroniques repose sur l'utilisation d'un réseau de capteurs à réactivité croisée donnant une « empreinte chimique » globale de l'échantillon à analyser. Bien que potentiellement adaptés à des mesures en abattoir, les nez électroniques présentent à ce jour un problème majeur de sélectivité. Par ailleurs, les capteurs utilisés dans les nez électroniques sont souvent sensibles à l'humidité qui devient alors un interférent majeur.

Du fait de l'absence d'un procédé physico-chimique de détection du scatol qui soit à la fois sensible, sélectif et adapté à une mise en oeuvre en abattoir, la majorité des filières d'abattage de porcs mâles entiers utilise actuellement un panel de « nez humains ». Le protocole consiste à chauffer un échantillon de tissu adipeux à l'aide d'un fer à souder ou d'un appareil équivalent, le plus souvent directement sur les carcasses, et de faire sentir les composés odorants ainsi libérés par des opérateurs entraînés spécifiquement à cette tâche. Cette méthode de détection est coûteuse et contraignante, peu agréable pour les opérateurs et se montre efficace uniquement quand un faible nombre de carcasses doit être trié, parce que les récepteurs olfactifs des opérateurs sont rapidement saturés. Par ailleurs, cette méthode souffre d'une grande variabilité dans les résultats en fonction des opérateurs, avec un taux de vrais positifs qui n'est que de 70 % seulement.

Compte-tenu de ce qui précède, les Inventeurs se sont fixé pour but de fournir un procédé physico-chimique qui permette de détecter la présence de scatol dans des échantillons de tissus adipeux de porcs avec une grande sensibilité et une haute spécificité de sorte à ce qu'il conduise à des résultats extrêmement fiables.

Ils se sont aussi fixé pour but que ce procédé permette de plus, si du scatol est présent dans les échantillons de tissus adipeux, de doser ce scatol si on le souhaite.

Ils se sont de plus fixé pour but que la mise en oeuvre de ce procédé puisse être réalisée dans des abattoirs et ce, quelles que soient les conditions de température et d'humidité qui y règnent, et que cette mise en oeuvre soit suffisamment simple et rapide pour être compatible avec les contraintes de cadence de tri de carcasses auxquelles les abattoirs sont soumis.

Ils se sont encore fixé pour but que la mise en oeuvre de ce procédé ait un coût qui n'impacte pas de manière significative le prix de vente ultérieur de la viande de porc.

Ils se sont en outre fixé pour but que ce procédé permette une traçabilité des échantillons de tissus adipeux de porcs analysés.

## Exposé de l'invention

Tous ces buts sont atteints par l'invention qui propose, en premier lieu, un procédé pour détecter la présence de scatol dans un échantillon d'un tissu adipeux de porc, lequel procédé est caractérisé en ce qu'il comprend au moins

les étapes consistant à :

a) soumettre un extrait organique de l'échantillon de tissu adipeux à une réaction d'électrochimioluminescence ;
b) mesurer l'intensité de la luminescence pendant l'étape a) et, si l'intensité de luminescence mesurée dépasse une valeur seuil prédéterminée, déduire la présence de scatol dans l'échantillon de tissu adipeux.

Ainsi, la détection du scatol par le procédé de l'invention est basée sur la technique d'électrochimioluminescence.

On rappelle que l'électrochimioluminescence (ou ECL), aussi appelée chimio-luminescence électrogénérée, est une technique qui consiste à initier un processus de luminescence par un transfert d'électrons se produisant directement à la surface d'une électrode de travail.

Différents mécanismes d'ECL existent.

Dans le cadre de l'invention, on préfère que le processus de luminescence soit initié par l'application d'un potentiel cathodique à une électrode de travail plongée dans l'extrait organique pour induire la formation d'ions superoxydes ( $O_2^{-\bullet}$ ) par réduction du dioxygène dissous dans cet extrait.

Auquel cas, si du scatol est présent dans l'extrait organique, la réduction de l'oxygène en ions superoxydes est suivie :

- de la formation de la base conjuguée du scatol selon la réaction :

$$\text{3-MIH} + O_2^{-\bullet} \rightarrow \text{3-MI}^- + HO_2^\bullet,$$

puis
- de la formation de radicaux hydroperoxydes selon la réaction :

$$O_2^{-\bullet} + HO_2^\bullet \rightarrow O_2 + HO_2^-.$$

L'application subséquente d'un potentiel anodique à l'électrode de travail permet alors d'oxyder la base conjuguée du scatol qui, une fois oxydée, va réagir avec les radicaux hydroperoxydes pour conduire à la formation de N-(2-acétylphényl)formamide à l'état excité, lequel, par désexcitation (ou, autrement dit, par retour à son état fondamental), émet une luminescence mesurable.

Il est à noter que ce mécanisme d'ECL du scatol a été étudié et décrit par T. Okajima et T. Ohsaka (cf. Journal of Electroanalytical Chemistry 2002, 523, 34-39). Toutefois, cette étude a été réalisée dans un contexte purement théorique, visant à appréhender les processus chimiques mis en jeu dans l'ECL du scatol en solution dans l'acétonitrile mais sans qu'une quelconque application pratique de ces processus soit envisagée et, notamment, une application à la détection du scatol dans des tissus adipeux de porcs.

Compte-tenu de ce qui précède, on préfère que l'extrait organique remplisse les conditions suivantes :

- comprendre un solvant organique aprotique ;
- être anhydre, c'est-à-dire comprendre au plus 1 % massiques d'eau ; et
- être électroconducteur, ce qui est rendu possible par la présence dans cet extrait d'un sel de fond.

Pour ce faire, l'invention propose de préparer préalablement l'extrait organique par :

i) séparation du gras présent dans l'échantillon de tissu adipeux des éléments non graisseux également présents dans l'échantillon de tissu adipeux, tels que résidus de peau, fibres musculaires, etc. ;
ii) déshydratation du gras présent dans l'échantillon de tissu adipeux, sachant qu'un tissu de porc comprend naturellement de l'ordre de 15 % massiques d'eau ;
iii) dissolution du gras obtenu à l'issue de l'étape ii) dans un solvant organique aprotique pour obtenir une solution organique et chauffage de la solution organique pendant un temps suffisant pour permettre l'extraction du scatol si celui-ci est présent ;
iv) dégraissage de la solution organique obtenue à l'issue de l'étape iii) ; et
v) ajout d'un sel de fond anhydre à la solution organique obtenue à l'issue de l'étape iv) ;

les étapes i) et ii) pouvant être réalisées successivement ou simultanément.

Conformément à l'invention, les étapes i) et ii) sont, de préférence, réalisées simultanément.

Pour ce faire, il est, par exemple, possible de chauffer l'échantillon de tissu adipeux à une température au moins égale à 100 °C et, de préférence, comprise entre 100 °C et 150 °C, et dans un récipient ouvert de sorte que l'eau présente dans l'échantillon de tissu adipeux puisse s'évacuer sous forme de vapeur d'eau. Ceci permet de liquéfier le gras présent dans l'échantillon de tissu adipeux et, ainsi, de faciliter sa séparation ultérieure des éléments non graisseux également présents dans cet échantillon qui, eux, vont rester à l'état solide, et, en même temps, de le déshydrater. Ceci permet en outre de dénaturer les protéines présentes dans l'échantillon de tissu adipeux qui vont ensuite précipiter lors de l'étape iii), ce qui participe à réduire la quantité d'espèces électro-actives qui seront présentes dans l'extrait organique.

En variante, il est également possible de soumettre l'échantillon de tissu adipeux à un chauffage à une température inférieure à 100°C avec un tirage sous vide.

La durée du chauffage sera notamment choisie en fonction de la masse de l'échantillon de tissu adipeux, de la forme, prédécoupée ou non, sous laquelle il se présente et s'il est prédécoupé, de la taille des morceaux de l'échantillon, de la configuration du récipient dans lequel est réalisé le chauffage, du mode de chauffage (sans ou avec micro-ondes par exemple), de l'utilisation ou non d'un tirage sous vide et de la température choisie pour réaliser le chauffage.

À l'étape iii), le gras obtenu à l'issue de l'étape précédente ou des deux étapes précédentes (si celles-ci sont réalisées simultanément) est dissous dans un solvant organique aprotique et la solution organique résultante est chauffée.

Dans ce qui précède et ce qui suit, le terme « aprotique », lorsqu'il est appliqué à un solvant organique, est pris dans son acceptation habituelle, à savoir qu'il désigne un solvant organique dont la molécule est exempte d'atome hydrogène acide, c'est-à-dire lié à un hétéroatome comme un atome d'azote, d'oxygène ou de soufre.

Conformément à l'invention, le solvant aprotique est avantageusement un solvant aprotique polaire, c'est-à-dire présentant un moment dipolaire non nul, comme l'acétonitrile, le diméthylsulfoxyde, le carbonate de propylène ou la $\gamma$-butyrolactone, préférence étant donnée à l'acétonitrile.

Le chauffage de l'étape iii) est avantageusement réalisé, sous agitation, à une température de 50 °C à 80 °C, pendant de 1 minute à 10 minutes, et ce, dans un récipient fermé pour éviter que le solvant organique aprotique ne s'évapore.

L'étape iv) est, elle, avantageusement réalisée en soumettant la solution organique obtenue à l'issue de l'étape iii) à une centrifugation pour casser l'émulsion s'étant formée au cours de l'étape iii) du fait de l'agitation, puis en maintenant cette solution organique à une température inférieure ou égale à 4°C mais supérieure à la température de solidification du solvant organique aprotique de sorte à figer la partie graisseuse de la solution organique mais sans que le solvant organique ne solidifie, et en éliminant ensuite la partie graisseuse ayant figé.

Le sel de fond, qui est ajouté à la solution organique obtenue à l'issue de l'étape iv), peut être choisi parmi de très nombreux sels étant entendu qu'il doit être, d'une part, soluble dans le solvant organique aprotique, et d'autre part, chimiquement et électro-chimiquement inerte pour ni ne perturber la réaction d'ECL, ni induire une réaction indésirable avec le scatol. Comme bien connu des électrochimistes, ce sel peut notamment être un tétrafluoroborate, un hexaflurorophosphate ou un perchlorate de tétraalkylammonium dans lequel le groupe alkyle comprend de 1 à 6 atomes de carbone, tel que le tétrafluoroborate de tétrabutylammonium ou l'hexafluorophosphate de tétrabutylammonium, ce type de sel présentant, en effet, une stabilité remarquable en milieu organique.

Par ailleurs, le sel de fond est ajouté à la solution organique obtenue à l'issue de l'étape iv) en une quantité telle que sa concentration dans l'extrait organique soit typiquement comprise entre 0,01 mol/L et 1 mol/L, de préférence entre 0,05 mol/L et 0,5 mol/L, et, mieux encore, égale à 0,1 mol/L.

Conformément à l'invention, il est possible d'ajouter à la solution organique obtenue, que ce soit à l'issue de l'étape iii), de l'étape iv) et/ou de l'étape v), un agent de séchage tel qu'un sel hygroscopique du type sulfate de sodium ou de magnésium anhydre, ou un tamis moléculaire (3 ou 4 angströms) pour compléter la déshydratation réalisée à l'étape ii).

Dans un mode de mise en oeuvre préféré du procédé de l'invention, l'extrait organique comprend, de plus, une base forte, auquel cas cette base forte est, de préférence, ajoutée à la solution organique obtenue à l'issue de l'étape v).

La présence d'une base forte dans l'extrait organique permet, en premier lieu, de déprotoner les acides organiques présents dans cet extrait. En effet, comme il sera montré ultérieurement, le scatol devient à plusieurs stades de la réaction d'ECL accepteur de protons. Or, tout extrait organique préparé à partir d'un échantillon d'un tissu adipeux de porc contient un certain nombre de composés donneurs de protons et, notamment, des acides organiques, lesquels sont essentiellement des acides gras solubles dans les solvants organiques que des opérations de dégraissage telles que celle conduite à l'étape iv) ne permettent pas d'éliminer. Il est donc souhaitable de neutraliser ces composés, ce qui est rendu possible par la présence d'une base forte dans l'extrait organique.

La présence d'une base forte dans l'extrait organique permet de plus d'assurer la disparition de toute trace d'eau résiduelle dans l'extrait organique.

Par base forte, on entend, dans le cadre de l'invention, une base pouvant déprotoner l'eau.

Cette base peut notamment être une base organique telle que la triéthylamine, l'imidazole, l'histidine, ou toute autre amine tertiaire, un alcoolate ou un amidure d'un métal alcalin tel que l'alcoolate ou l'amidure de sodium, un hydrure d'un métal alcalin ou alcalino-terreux tel que l'hydrure de sodium, l'hydrure de lithium, l'hydrure de potassium ou l'hydrure de rubidium, un nitrure d'un métal alcalin ou alcalino-terreux comme le nitrure de sodium.

Préférence est donnée à une base forte présentant le pKa le plus élevé possible.

Une base forte particulièrement préférée est l'hydrure de sodium qui, outre d'avoir un acide conjugué au pKa élevé, a l'avantage d'être peu coûteux, facile à se procurer et dont la réaction avec les espèces acides conduit à leur équivalent sodé - lequel précipite et peut donc être éliminé - avec une libération sous forme gazeuse de l'hydrogène présent dans l'extrait organique. À titre d'exemple, la réaction de l'hydrure de sodium avec l'eau encore potentiellement présente dans l'extrait organique est la suivante :

$$H_2O + NaH \rightarrow NaOH \text{ (qui précipite)} + H_2 \text{ (gazeux).}$$

Comme connu en soi, la réaction d'ECL est, de préférence, réalisée dans une cellule électrochimique, les termes « cellule électrochimique » désignant ici l'ensemble formé par un contenant de type cuvette ou analogue, dans lequel est placé l'extrait organique pour la réaction d'ECL, et au moins deux électrodes, à savoir une électrode de travail et une contre-électrode.

Comme précédemment indiqué, la réaction d'ECL qui est privilégiée dans le cadre de l'invention comprend l'application d'un potentiel cathodique à l'électrode de travail de la cellule électrochimique pour induire la formation des ions superoxydes, suivie de l'application d'un potentiel anodique à cette même électrode pour induire l'oxydation de la base conjuguée du scatol si celui-ci est présent dans l'extrait organique.

Ceci peut être réalisé selon différents protocoles électrochimiques et, notamment, par :

- un balayage en potentiel, auquel cas on applique à l'électrode de travail un balayage vers les potentiels négatifs puis un balayage vers les potentiels positifs ;
- un saut de potentiel, auquel cas on applique à l'électrode de travail un potentiel négatif constant, pendant une durée suffisante pour saturer la surface de cette électrode en ions superoxydes, puis un potentiel positif constant, également pendant une durée suffisante pour saturer la surface de l'électrode de travail en scatol oxydé ; ou
- par une série d'impulsions de potentiel alternativement cathodique et anodique.

Il va de soi que les paramètres électrochimiques doivent être adaptés, notamment en fonction de la configuration de la cellule électrochimique utilisée, de la nature de l'électrode de travail et de la contre-électrode utilisées, c'est-à-dire du matériau d'électrode qui constitue ces électrodes, de la manière dont l'extrait organique a été préparé et, donc, de sa composition et de sa viscosité. Si une électrode de référence ou de pseudo-référence est utilisée, les paramètres électrochimiques doivent également être adaptés en fonction de la nature de cette électrode.

À titre d'exemples, pour des extraits organiques préparés comme décrit ci-avant et des cellules électrochimiques munies d'une électrode de travail et d'une contre-électrode en diamant dopé au bore ainsi que d'une électrode de pseudo-référence en platine, d'excellents résultats ont été obtenus en appliquant à l'électrode de travail :

- dans le cas d'un balayage en potentiel : un balayage allant de 0 V à un potentiel négatif compris entre -1,5 V et -2,5 V (*vs* Pt), par exemple de -2 V (*vs* Pt), puis un balayage allant de ce potentiel négatif à un potentiel positif supérieur ou égal à +0,5 V (*vs* Pt), par exemple de +0,8 V (*vs* Pt), avec une vitesse de balayage constante comprise entre 10 mV/s et 100 mV/s, par exemple de 50 mV/s ;
- dans le cas d'un saut de potentiel : un potentiel négatif constant inférieur à -1,5 V (*vs* Pt), par exemple de -2 V (*vs* Pt), pendant de 0,1 seconde à 45 secondes, puis un potentiel positif constant supérieur à 0,5 V (*vs* Pt), par exemple de +1 V (*vs* Pt), pendant de 10 secondes à 30 secondes ; et
- dans le cas d'impulsions de potentiel alternativement cathodique et anodique : un potentiel négatif inférieur à -1,5 V (*vs* Pt), par exemple de -2 V (*vs* Pt) suivi d'un potentiel positif supérieur à 0,5 V (*vs* Pt), par exemple de +1 V (*vs* Pt), à une fréquence allant de 0,5 hertz à 5 hertz, par exemple d'1 hertz.

Le choix du contenant de la cellule électrochimique n'est pas critique en soi.

Toutefois, il est souhaitable que ce contenant soit en un matériau résistant aux solvants organiques, aux milieux salins et, si une base forte est utilisée, aux milieux alcalins.

Par ailleurs, il convient que ce contenant soit en un matériau optiquement transparent dans la gamme des longueurs d'onde d'émission de la luminescence ou qu'il présente au moins une paroi en un matériau présentant une telle transparence si la détection des photons émis est réalisée par un détecteur optique situé en vis-à-vis de l'une de ses parois.

Le choix des électrodes de la cellule électrolytique n'est pas critique non plus.

Si la réaction d'ECL est basée sur la formation d'ions superoxydes, alors l'électrode de travail peut être constituée de tout matériau d'électrode permettant la formation de tels ions comme du carbone (graphite, carbone vitreux, diamant dopé, par exemple au bore ou à l'azote, etc), un métal noble (or, platine, palladium, iridium, etc) ou un alliage de métaux nobles.

La contre-électrode peut être constituée d'un matériau d'électrode différent ou du même matériau d'électrode que celui qui constitue l'électrode de travail.

Dans le cadre de l'invention, on préfère que l'électrode de travail et la contre-électrode soient en diamant dopé, notamment au bore, en raison de ce que ce matériau est hautement conducteur, présente une très grande stabilité, une résilience naturelle à l'encrassement due à la forte densité atomique du diamant. Cette résilience à l'encrassement est particulièrement intéressante compte-tenu que l'extrait organique peut comprendre un certain nombre de composés issus du tissu adipeux de porc, dont des acides gras, qui peuvent encrasser rapidement la surface des électrodes. De plus, en cas d'encrassement, ce type d'électrode peut être aisément décrassé par voie électrochimique, par exemple par le procédé décrit dans le brevet US 9,121,107. Enfin, ce type d'électrode présente une grande fenêtre de potentiel qui permet d'appliquer des potentiels élevés sans venir électrolyser le solvant présent dans l'extrait organique.

Si une électrode de référence est utilisée, alors celle-ci peut notamment être une électrode au calomel saturé (ECS) ou une électrode au chlorure d'argent (Ag/AgCl), éventuellement à double jonction, telles que traditionnellement utilisées en électrochimie. Toutefois, dans le cadre de l'invention, on préfère utiliser une électrode de pseudo-référence en un métal noble tel que le platine parce que ce type d'électrode peut également être aisément décrassé, par exemple par passage à la flamme.

Avantageusement, la cellule électrochimique (i.e. contenant et électrodes) sont réalisés dans des matériaux à bas coûts (contenant en plastique, électrodes à pâte de carbone, etc) de sorte à être à usage unique, ce qui permet, d'une part, de s'affranchir des problèmes d'encrassement des électrodes et, d'autre part, d'assurer une traçabilité des échantillons de tissus adipeux analysés, par exemple en référençant chaque cellule électrochimique par rapport à une carcasse de porc.

Quant au détecteur optique, il peut s'agir d'un photomultiplicateur couplé à une photocathode en bialkali, super-bialkali ou ultra-bialkali, d'une photodiode à avalanche, d'un photomultiplicateur à photocathode en silicium, d'un spectromètre et, notamment, un spectrofluorimètre, d'un détecteurà capteur CCD (de « Charged Coupled Device »), d'un détecteur à capteur CMOS (de « Complementary Metal-Oxide-Semiconductor »), etc.

Conformément à l'invention, la valeur seuil est, de préférence, au moins égale à 150 % de la valeur moyenne de l'intensité de la luminescence correspondant au bruit de fond du détecteur optique.

Comme précédemment indiqué, le procédé de l'invention permet aussi, si du scatol est présent dans l'extrait organique, d'en déterminer la teneur.

Ainsi, si la présence de scatol a été déduite à l'étape b), le procédé comprend avantageusement de plus une quantification du scatol présent dans l'extrait organique par comparaison de l'intensité maximale de luminescence mesurée au cours de l'étape b) avec une courbe d'étalonnage, cette quantification étant réalisée au cours de l'étape b) ou postérieurement à l'étape b).

Le procédé de l'invention permet de détecter la présence de scatol dans un tissu adipeux de porc avec une grande sensibilité puisqu'une limite de détection autour de 20 nmol/L de scatol a pu être obtenue.

Le seuil de rejet d'une viande de porc par le consommateur est fixé à environ 0,2 $\mu$g de scatol par gramme de tissu adipeux, ou gras brut, ce qui correspond, dans l'hypothèse d'une extraction complète du scatol présent dans 1g de tissu adipeux dans 1 mL d'un solvant organique à une concentration de scatol de 1,53 $\mu$mol/L.

Il en résulte que la limite de détection du scatol par le procédé de l'invention est autour de 3 ordres de grandeur en dessous de la concentration du scatol devant être détectée.

Par ailleurs, comme démontré par les expérimentations rapportées ci-après, la détection du scatol par le procédé de l'invention est hautement spécifique puisque d'autres composés indoliques présents dans les tissus adipeux de porcs comme l'indole ne sont pas détectés avec ce procédé.

L'absence d'excitation photonique (par UV, lumière blanche filtrée ou autre) dans la cellule d'électrolyse empêche toute fluorescence spontanée d'interférents potentiels et permet ainsi d'obtenir un rapport signal/bruit très élevé.

En outre, le procédé de l'invention est robuste, tant par l'équipement qu'il nécessite que dans sa mise en oeuvre, simple, rapide et peu coûteux.

Il est donc particulièrement bien adapté à une mise en oeuvre en abattoir.

Aussi, l'invention a-t-elle aussi pour objet un procédé de tri de carcasses de porcs mâles entiers, qui est caractérisé en ce qu'il comprend la mise en oeuvre d'un procédé de détection du scatol tel que précédemment défini.

D'autres caractéristiques et avantages de l'invention ressortiront du complément de description qui suit, qui se rapporte à des expérimentations ayant permis de valider l'invention et qui est donné en référence aux figures annexées.

Il va de soi toutefois que ce complément de description n'est donné qu'à titre d'illustration de l'objet de l'invention et ne doit en aucun cas être interprété comme une limitation de cet objet.

## Brève description des figures

La figure 1 représente schématiquement une première cellule électrochimique ayant été utilisée dans les expérimentations décrites ci-après, vue en coupe.

La figure 2 représente schématiquement une deuxième cellule électrochimique ayant été utilisée dans les expérimentations décrites ci-après, vue de 3/4.

La figure 3 illustre le voltammogramme obtenu en soumettant un extrait organique d'un échantillon d'un tissu adipeux de porc non contaminé au scatol, ayant été additionné de scatol de synthèse, à une réaction d'ECL par balayage en potentiel.

La figure 4 illustre la luminescence mesurée simultanément à l'enregistrement du voltammogramme montré sur la figure 3.

La figure 5 illustre le chronoampérogramme obtenu en soumettant un extrait organique d'un échantillon d'un tissu adipeux de porc non contaminé au scatol, ayant été additionné de scatol de synthèse, à une réaction d'ECL par saut de potentiel.

La figure 6 illustre la luminescence mesurée simultanément à l'enregistrement du chronoampérogramme montré sur la figure 5.

La figure 7 illustre la réponse en courant obtenue en soumettant une solution comprenant du scatol de synthèse et de l'hexafluorophosphate de tétrabutylammonium dans l'acétonitrile à une réaction d'ECL par application d'une répétition d'impulsions de tensions alternativement cathodiques et anodiques.

La figure 8 illustre la luminescence mesurée simultanément à l'enregistrement de la réponse en courant montrée sur la figure 7.

La figure 9 illustre la luminescence mesurée en soumettant une solution comprenant du scatol de synthèse et de l'hexafluorophosphate de tétrabutylammonium dans l'acétonitrile à une réaction d'ECL par balayage en potentiel.

La figure 10 illustre la luminescence mesurée en soumettant une solution comprenant de l'indole et de l'hexafluorophosphate de tétrabutylammonium dans l'acétonitrile à une réaction d'ECL par balayage en potentiel.

La figure 11 illustre la luminescence mesurée en soumettant un extrait organique d'un échantillon d'un tissu adipeux de porc contaminé au scatol à une réaction d'ECL par saut de potentiel.

La figure 12 illustre la luminescence mesurée en soumettant un extrait organique d'un échantillon d'un tissu adipeux de porc non contaminé au scatol à une réaction d'ECL par saut de potentiel.

La figure 13 illustre deux courbes étalons réalisées en soumettant des extraits organiques d'échantillons d'un tissu adipeux de porc non contaminé au scatol mais ayant été additionnés de scatol de synthèse à hauteur de 0,1 μmol/L à 5 μmol/L (courbe 1) et des solutions comprenant de 0,1 μmol/L à 5 μmol/L de scatol de synthèse et de l'hexafluorophosphate de tétrabutylammonium dans l'acétonitrile (courbe 2) à une réaction d'ECL par saut de potentiel.

Sur la figure 3, l'axe des ordonnées correspond à l'intensité, notée I et exprimée en microampères (μA), du courant mesuré à l'électrode de travail, tandis que l'axe des abscisses correspond au potentiel, noté V et exprimé en volts (V) par rapport au potentiel de l'électrode de référence, appliqué à l'électrode de travail.

Sur les figures 5 et 7, l'axe des ordonnées correspond à l'intensité, notée I et exprimée en milliampères (mA) sur la figure 5 et en microampères (μA) sur la figure 7, du courant mesuré à l'électrode de travail tandis que l'axe des abscisses correspond au temps, noté t et exprimé en secondes (s).

Sur les figures 4, 6, 8 à 12, l'axe des ordonnées correspond au nombre de coups émis, noté Ne et exprimé en unités arbitraires (u.a.), tandis que l'axe des abscisses correspond au temps, noté t et exprimé en secondes (s).

Sur la figure 13, l'axe des ordonnées correspond au nombre de coups émis, noté Ne et exprimé en unités arbitraires (u.a.), tandis que l'axe des abscisses correspond à la concentration du scatol, notée [C] et exprimée en micromoles/L (μmol/L).

## Exposé détaillé de modes de mise en oeuvre particuliers

Les expérimentations qui sont décrites ci-après sont réalisées en utilisant :

- soit des extraits organiques d'échantillons de tissus adipeux de porcs, contaminés ou non en scatol ;
- soit des solutions dites ci-après « solutions standards » et comprenant du scatol de synthèse dans l'acétonitrile.

## I - Préparation des extraits organiques d'échantillons de tissus adipeux de porcs et des solutions standards

### I.1 - Extraits organiques :

Les extraits organiques d'échantillons de tissus adipeux de porc, contaminés ou non en scatol, sont préparés en suivant, pour chaque extrait, le protocole opératoire suivant.

Un échantillon d'un tissu adipeux d'un porc, coupé finement, est placé dans un bécher en verre et porté à une température de 120 °C pendant 1 heure. Pendant ce chauffage, le bécher n'est pas couvert pour laisser s'échapper la vapeur d'eau. La taille de l'échantillon de tissu adipeux est choisie de sorte qu'au moins 5 mL de gras fondu soit recueillis à la fin de cette étape.

Puis, 5 mL de ce gras fondu sont transférés dans un tube à essai dans lequel sont ajoutés 5 mL d'acétonitrile (pureté : 99,8 %). Le tube à essai est fermé, puis le mélange est porté à une température de 70 °C pendant 10 minutes. Une solution est obtenue qui est agitée à l'aide d'un agitateur vortex pendant 5 minutes, puis centrifugée à 4000 rpm pendant 15 minutes pour obtenir une phase graisseuse et une phase solvant.

Le tube est ensuite placé dans un congélateur à -18 °C pendant au moins 2 heures pour figer la phase graisseuse. La phase solvant est récupérée et transférée dans un nouveau tube à essai.

À cette phase, sont ajoutés 100 mg de sulfate de magnésium pour finir de l'assécher puis 0,385 mg d'hexafluorophosphate de tétrabutylammonium (TBAHFF) pour lui conférer une concentration en sel de fond de 0,1 mol/L.

Les extraits ainsi préparés sont conservés au réfrigérateur jusqu'à leur usage.

### I.2 - Solutions standards :

Les solutions standards sont préparées en dissolvant, sous agitation, du scatol de synthèse dans l'acétonitrile à une concentration allant de 0,1 $\mu$mol/L à 5 $\mu$mol/L, puis en ajoutant aux solutions résultantes du TBAHFF pour leur conférer une concentration en sel de fond de 0,1 mol/L.

## II - Détection du scatol

### II.1 - Appareillage expérimental :

Les réactions d'ECL sont réalisées au moyen des deux cellules électrochimiques, respectivement notées 10 et 20, qui sont schématiquement illustrées sur les figures 1 et 2, et d'un potentiostat PalmSens4™ (PALMSENS).

La cellule 10 comprend une cuvette de spectroscopie UV/visible à usage unique, en polystyrène, dont la partie inférieure est à section quadrangulaire et dans laquelle deux électrodes 11 et 12 en diamant dopé au bore, servant respectivement d'électrode de travail et de contre-électrode, sont positionnées sur deux parois opposées de cette partie inférieure en sorte que ces électrodes se font donc face. Les électrodes 11 et 12 présentent une aire de surface de 1 $cm^2$.

La cellule 20 comprend, elle, une cuvette de forme parallélépipédique dans laquelle deux électrodes 21 et 22 en diamant dopé au bore, servant respectivement d'électrode de travail et de contre-électrode, sont positionnées sur les deux parois opposées de cette cuvette qui présentent la plus grande surface mais de manière décalée l'une par rapport à l'autre en sorte que ces électrodes sont disposées parallèlement l'une à l'autre mais sans se faire face. Les électrodes 21 et 22 présentent une aire de surface de 4 $cm^2$.

Les cellules 10 et 20 sont de plus munies d'un fil de platine, respectivement 13 et 23, servant d'électrode de pseudo-référence.

La détection des photons émis au cours des réactions d'ECL est réalisée dans la gamme de longueurs d'onde allant de 450 nm à 550 nm, au moyen d'un spectrofluorimètre Fluoromax™ 4 ou 4P (HORIBA JOBIN YVON) qui permet de suivre en temps réel l'évolution de la luminescence grâce à un logiciel intégré.

Pendant les réactions d'ECL, les cellules électrochimiques sont placées dans le noir absolu pour limiter le bruit de fond du spectrofluorimètre.

### II.2 - Déprotonation des acides organiques :

Les mesures du scatol dans les extraits organiques sont réalisées après déprotonation des acides organiques présents dans ces extraits.

Cette déprotonation est obtenue en ajoutant à chaque extrait de l'hydrure de sodium (NaH) sous la forme d'une dispersion à 60 % de NaH dans l'huile minérale (CAS : 7646-69-7), à raison de 60 mg de cette dispersion pour 5 mL d'extrait. Lors de cet ajout, un bullage est observé, correspondant à un dégagement de dihydrogène. L'extrait est ensuite laissé au repos pendant 15 minutes au cours desquelles un précipité se forme, correspondant à la saponification des acides organiques présents dans l'extrait. Ce précipité est éliminé.

**II.3** - **Vérification de la possibilité de réaliser la réaction d'ECL selon différents protocoles électrochimiques** :

Comme précédemment indiqué, la réaction d'ECL du scatol qui est privilégiée dans le cadre de l'invention est une réaction induite par l'application d'un potentiel cathodique à l'électrode de travail d'une cellule électrochimique pour induire la formation d'ions superoxydes, dans un extrait organique obtenu à partir d'un échantillon d'un tissu adipeux de porc, par réduction du dioxygène dissous dans cet extrait.

Cette réaction peut être décomposée en les étapes suivantes :

- application d'un potentiel cathodique :

(1) formation d'ions superoxydes par réduction du dioxygène dissous :

$$\mathbf{O_2} + \boldsymbol{e^-} \leftrightarrow \mathbf{O_2^{-\bullet}}$$

(2) formation de la base conjuguée du scatol en présence des ions superoxydes :

$$\text{3-MIH} + \mathbf{O_2^{-\bullet}} \rightarrow \text{3-MI}^- + \mathbf{HO_2^{\bullet}}$$

(3) formation de radicaux hydroperoxyles :

$$\mathbf{O_2^{-\bullet}} + \mathbf{HO_2^{\bullet}} \rightarrow \mathbf{O_2} + \mathbf{HO_2^{-}}$$

- application d'un potentiel anodique :

(4) oxydation du scatol :

$$\textbf{3-MI}^- - \boldsymbol{e^-} \rightarrow \textbf{3-MI}^\bullet$$

avec :

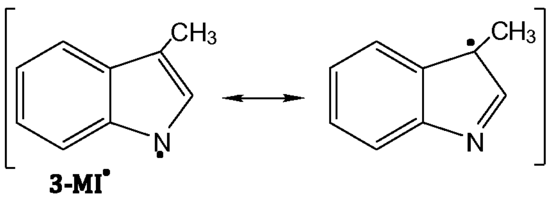

**3-MI•**

(5) couplage radicalaire du scatol oxydé avec $\mathbf{HO_2^{\bullet}}$ impliquant la formation d'un composé intermédiaire comportant un groupement 1,2-dioxétane puis de N-(2-acétylphényl)formamide dans un état excité :

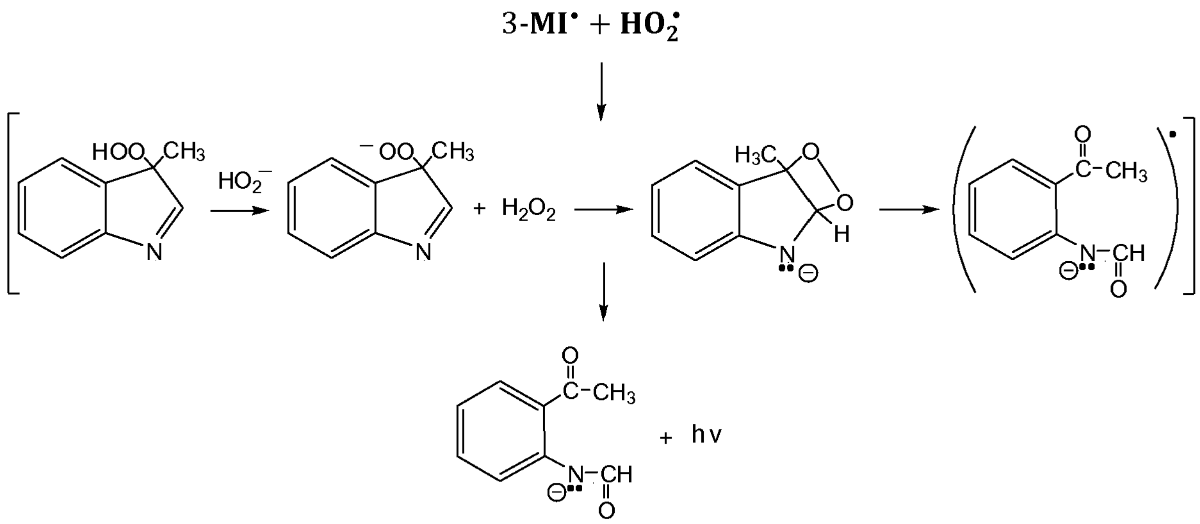


Pour obtenir cette réaction, trois protocoles électrochimiques sont testés :

- un balayage en potentiel (voltammétrie cyclique) ;
- un saut de potentiel (chronoampérométrie) ; et
- une application répétée d'impulsions de tensions alternativement cathodiques et anodiques.

* *Balayage en potentiel :*

Pour le balayage en potentiel, on utilise :

- un extrait organique d'un échantillon d'un tissu adipeux de porc non contaminé en scatol auquel a été ajouté du scatol synthétique à une concentration de 5 µmol/L, et
- la cellule électrochimique 10 montrée sur la figure 1.

Après avoir introduit l'extrait organique dans la cellule électrochimique, on applique à l'électrode de travail 11 de cette cellule un balayage en potentiel de 0 à -2 V puis de -2 V à +0,8 V *(versus* l'électrode de référence 13) avec une vitesse de balayage de 50 mV/s.
La mesure de la luminescence est lancée dès le début du balayage en potentiel.
Le voltammogramme ainsi obtenu et la luminescence ainsi mesurée sont illustrés sur les figures 3 et 4 respectivement.
Comme le montre la figure 4, un pic de luminescence est observé dans la zone de potentiel anodique du balayage, qui peut être relié à la présence de scatol dans l'extrait organique et dont l'amplitude peut, elle, être reliée à la quantité de scatol présent dans cet extrait.

* *Saut de potentiel :*

Pour le saut de potentiel, on utilise :

- un extrait organique d'un échantillon d'un tissu adipeux de porc non contaminé en scatol auquel a été ajouté du scatol synthétique à une concentration de 5 µmol/L, et
- la cellule électrochimique 20 montrée sur la figure 2.

Après avoir introduit l'extrait organique dans la cellule électrochimique, on applique à l'électrode de travail 21 de cette cellule un potentiel de -2 V *(versus* l'électrode de référence 23) pendant 30 secondes, puis de +1 V *(versus* l'électrode de référence 23) pendant encore 30 secondes.
Là également, la mesure de la luminescence est lancée dès le début de l'application d'un potentiel à l'électrode de travail.
Le chronoampérogramme ainsi obtenu et la luminescence ainsi mesurée sont illustrés sur les figures 5 et 6 respectivement.
Comme le montre la figure 6, un pic intense de luminescence est observé lors du passage du premier potentiel

au second potentiel, ce pic pouvant, là également, être relié à la présence de scatol dans l'extrait organique et dont l'amplitude peut, elle, être reliée à la quantité de scatol présent dans cet extrait.

* *Régime impulsionnel :*

Le test consistant à appliquer à l'électrode de travail une répétition d'impulsions de tensions alternativement cathodiques et anodiques est réalisé en utilisant :

- une solution standard à 5 μmol/L de scatol, et
- la cellule électrochimique 20 montrée sur la figure 2.

Après avoir introduit la solution standard dans la cellule électrochimique, on applique à l'électrode de travail 21 de cette cellule un potentiel de -2 V (*versus* l'électrode de référence 23) suivi d'un potentiel de +1 V (*versus* l'électrode de référence 23) de manière répétée à une fréquence d'1 Hz.

Là également, la mesure de la luminescence est lancée dès le début de l'application d'un potentiel à l'électrode de travail.

La réponse en courant de l'électrode de travail ainsi enregistrée et la luminescence ainsi mesurée sont illustrées sur les figures 7 et 8 respectivement.

Comme le montre la figure 8, l'émission d'un signal de luminescence variable mais d'intensité moyenne aux alentours de 14 000 coups est observé pendant toute la durée de l'application du régime impulsionnel.

Un autre test est conduit dans les mêmes conditions expérimentales à ceci qu'une fréquence de 5 Hz est utilisée en lieu et place d'1 Hz. Une réponse similaire d'ECL est obtenue mais avec une intensité moyenne de luminescence d'environ 8 000 coups, soit plus faible que la précédente.

**II.4** - **Vérification du caractère spécifique de la détection du scatol** :

Deux expérimentations sont réalisées, à savoir :

- une première expérimentation qui consiste à soumettre une série statistiquement significative d'extraits organiques d'échantillons de tissus adipeux de porcs non contaminés en scatol (l'absence de scatol dans ces extraits ayant été vérifiée par chromatographie en phase gazeuse couplée à une spectrométrie de masse ou GC/MS, laquelle permet de doser le scatol jusqu'à la concentration de 0,03 μg/g de gras) à une réaction d'ECL et à mesurer la luminescence émise pendant cette réaction ;
- une deuxième expérimentation qui consiste à soumettre à une réaction d'ECL, d'une part, des solutions standards à 5 μmol/L de scatol, et, d'autre part, des solutions comprenant 5 μmol/L d'indole et 0,1 mol/L de TBAHFF dans l'acétonitrile, dans les mêmes conditions opératoires et à mesurer la luminescence émise pendant cette réaction.

À cet égard, on rappelle que le scatol et l'indole ne diffèrent structurellement l'un de l'autre qu'en ce que le cycle pyrrole du scatol est porteur d'un groupe méthyle contrairement au cycle pyrrole de l'indole.

La première expérimentation est réalisée dans la cellule électrochimique 10 montrée sur la figure 1 et par application d'un saut de potentiel, comme décrit au point II.3 ci-avant.

La deuxième expérimentation est réalisée dans la cellule électrochimique 20 montrée sur la figure 2 et par application d'un balayage en potentiel, comme également décrit au point II.3 ci-avant.

Aucun signal significatif de luminescence n'est détecté au cours de la première expérimentation, la luminescence mesurée étant du type de celle montrée sur la figure 12.

Comme le montrent les figures 9 et 10 qui représentent des exemples de la luminescence mesurée au cours de la deuxième expérimentation pour respectivement une solution standard de scatol et une solution d'indole, un signal intense de luminescence est observé pour la solution standard de scatol alors qu'aucun signal significatif de luminescence n'est observé pour la solution d'indole, confirmant ainsi le caractère très spécifique de la détection du scatol par le procédé de l'invention.

**II.5** -**Tests de détection du scatol en aveugle :**

Des tests de détection du scatol sont réalisés en soumettant des extraits organiques d'échantillons d'un tissu adipeux de porc contaminé en scatol (0,27 μg de scatol/g de tissu adipeux) et des extraits organiques d'échantillons d'un tissu adipeux de porc non contaminé en scatol à une réaction d'ECL dans la cellule électrochimique 20 montrée sur la figure 2 et par application d'un saut de potentiel comme décrit au point II.3 ci-avant et en mesurant la luminescence émise pendant cette réaction.

La contamination et l'absence de contamination des extraits organiques ont été préalablement vérifiées par GC/MS.

Les tests sont réalisés en aveugle, c'est-à-dire que l'expérimentateur ne sait pas, lorsqu'il soumet un extrait à une réaction d'ECL, si cet extrait est ou non contaminé en scatol.

La figure 11 montre un signal de luminescence tel que typiquement observé pour un extrait organique d'un échantillon de tissu adipeux de porc contaminé en scatol tandis que la figure 12 montre l'absence de signal significatif de luminescence telle qu'observée pour un extrait organique d'un échantillon de tissu adipeux de porc non contaminé en scatol.

**II.6** - **Courbes d'étalonnage :**

Afin de vérifier la possibilité de quantifier le scatol présent dans un extrait organique d'un tissu adipeux de porc, des courbes d'étalonnage sont réalisées en soumettant :

- d'une part, de solutions standards de scatol comprenant de 0,1 μmol/L à 5 μmol/L de scatol ; et
- d'autre part, des extraits organiques d'échantillons d'un tissu adipeux de porc non contaminé en scatol mais additionnés de scatol de synthèse à hauteur de 0,1 μmol/L à 5 μmol/L ;

à une réaction d'ECL et en mesurant l'intensité maximale de la luminescence émise pendant cette réaction.

La réaction d'ECL est réalisée dans la cellule électrochimique 10 montrée sur la figure 1 et en appliquant à l'électrode de travail 11 de cette cellule un potentiel de -2 V (*versus* l'électrode de référence 13) pendant 30 secondes, puis de +1 V (*versus* l'électrode de référence 13) pendant encore 30 secondes.

Les courbes d'étalonnage ainsi obtenues sont illustrées sur la figure 13, la courbe 1 correspondant aux extraits organiques additionnés de scatol de synthèse et la courbe 2 correspondant aux solutions standards de scatol.

Cette figure montre, pour les deux courbes, une relation quasi linéaire entre l'amplitude des pics de luminescence et la concentration en scatol.

La pente de la courbe 1 est environ deux fois plus faible que celle de la courbe 2, ce qui signifie que la sensibilité de détection du scatol dans le cas des extraits organiques additionnés de scatol de synthèse est environ deux fois plus faible que dans le cas des solutions standards de scatol.

Ceci s'explique expérimentalement, en premier lieu, par le fait que les extraits organiques additionnés de scatol de synthèse réabsorbent 30 % des photons émis (mesure effectuée par spectroscopie UV/visible). La perte des 20 % de signal restants est vraisemblablement due au fait que les extraits organiques additionnés de scatol de synthèse présentent une viscosité plus élevée que les solutions standards de scatol de synthèse et donc une mobilité ionique plus faible qui ralentit les processus de diffusion régissant les processus électrochimiques.

Toutefois, ces deux phénomènes (réabsorption des photons et augmentation de la viscosité) peuvent être facilement corrigés à partir, d'une part, du coefficient d'absorption de l'extrait organique analysé à la longueur d'onde d'intérêt (utilisation de la valeur moyenne qui a peu de risque de varier significativement d'un extrait organique à l'autre ou, le cas échéant, mesure réalisée en parallèle sur chaque extrait organique), et, d'autre part, des coefficients de diffusion des espèces ioniques mises en jeu dans l'extrait organique analysé que l'on aura évalués préalablement.

**Références citées**

K. Verplanken et al, Journal of Chromatography A 2016, 1462, 124-133
M. P. Aguilar-Caballos et al, Analytica Chimica Acta 2002, 460, 271-277
WO-A-83/00928
EP-A-2 966 441
X. Liu et al, Meat Science 2016, 116, 133-139
T. Okajima et T. Ohsaka, Journal of Electroanalytical Chemistry 2002, 523, 34-39 US-A-9,121,107

**Revendications**

1. Procédé pour détecter la présence de scatol dans un échantillon d'un tissu adipeux de porc, **caractérisé en ce qu'**il comprend au moins les étapes consistant à :

   a) soumettre un extrait organique de l'échantillon de tissu adipeux à une réaction d'électrochimioluminescence ;

b) mesurer l'intensité de la luminescence pendant l'étape a) et, si l'intensité de luminescence mesurée dépasse une valeur seuil prédéterminée, déduire la présence de scatol dans l'échantillon de tissu adipeux.

**2.** Procédé selon la revendication 1, dans lequel l'extrait organique comprend : (1) un solvant organique aprotique, moins de 1 % massiques d'eau et (3) un sel de fond.

**3.** Procédé selon la revendication 2, qui comprend, préalablement à l'étape a), une préparation de l'extrait organique, laquelle préparation comprend les étapes consistant à :

i) séparer le gras présent dans l'échantillon de tissu adipeux des éléments non graisseux également présents dans l'échantillon de tissu adipeux ;
ii) déshydrater le gras présent dans l'échantillon de tissu adipeux ;
iii) dissoudre le gras obtenu à l'issue de l'étape ii) dans un solvant organique aprotique pour obtenir une solution organique et chauffer la solution organique ;
iv) dégraisser la solution organique obtenue à l'issue de l'étape iii) ; et
v) ajouter un sel de fond anhydre à la solution organique obtenue à l'issue de l'étape iv) ;

les étapes i) et ii) pouvant être réalisées successivement ou simultanément.

**4.** Procédé selon la revendication 3, dans lequel les étapes i) et ii) sont réalisées simultanément.

**5.** Procédé selon la revendication 4, dans lequel les étapes i) et ii) comprennent un chauffage de l'échantillon du tissu adipeux à une température de 100 °C à 150 °C dans un récipient ouvert, ou à une température inférieure à 100 °C avec un tirage sous vide.

**6.** Procédé selon l'une quelconque des revendications 2 à 5, dans lequel le solvant organique est l'acétonitrile, le diméthylsulfoxyde, le carbonate de propylène ou la $\gamma$-butyrolactone, de préférence l'acétonitrile.

**7.** Procédé selon l'une quelconque des revendications 2 à 6, dans lequel Le chauffage de l'étape iii) est réalisé dans un récipient fermé, à une température de 50° C à 80° C, pendant de 10 minutes à 30 minutes et sous agitation.

**8.** Procédé selon l'une quelconque des revendications 2 à 7, dans lequel l'étape iv) comprend une centrifugation de la solution organique obtenue à l'issue de l'étape iii), puis un figement de la partie graisseuse de la solution organique par maintien de la solution organique à une température inférieure ou égale à 4°C mais supérieure à la température de solidification du solvant organique aprotique, et une élimination de la partie graisseuse ayant figé.

**9.** Procédé selon l'une quelconque des revendications 2 à 8, dans lequel le sel de fond est un tétrafluoroborate, un hexaflurorophosphate ou un perchlorate de tétraalkylammonium dans lequel le groupe alkyle comprend de 1 à 4 atomes de carbone, de préférence le tétrafluoroborate de tétrabutylammonium ou l'hexafluorophosphate de tétrabutylammonium.

**10.** Procédé selon l'une quelconque des revendications 2 à 9, dans lequel l'extrait organique comprend de 0,01 mol/L à 1 mol/L, de préférence de 0,05 mol/L à 0,5 mol/L et, mieux encore, 0,1 mol/L de sel de fond.

**11.** Procédé selon l'une quelconque des revendications 2 à 10, dans lequel l'extrait organique comprend de plus une base forte.

**12.** Procédé selon l'une quelconque des revendications 1 à 11, dans lequel l'étape a) est réalisée dans une cellule électrochimique comprenant au moins une électrode de travail et une contre-électrode, et la réaction d'électrochimioluminescence comprend l'application à l'électrode de travail d'un potentiel cathodique suivi d'un potentiel anodique.

**13.** Procédé selon la revendication 12, dans lequel l'application à l'électrode de travail d'un potentiel cathodique suivi d'un potentiel anodique est réalisée par balayage en potentiel, par saut de potentiel ou par une série d'impulsions de potentiel alternativement cathodique et anodique.

**14.** Procédé selon l'une quelconque des revendication 1 à 13, dans lequel, la présence de scatol ayant été déduite à l'étape b), on réalise de plus une quantification du scatol présent dans l'extrait organique par comparaison de

l'intensité maximale de luminescence mesurée au cours de l'étape b) avec une courbe d'étalonnage, la quantification étant réalisée au cours de l'étape b) ou postérieurement à l'étape b).

**15.** Procédé de tri de carcasses de porcs mâles entiers, **caractérisé en ce qu'**il comprend la mise en oeuvre d'un procédé selon l'une quelconque des revendications 1 à 14.

## Patentansprüche

**1.** Verfahren zur Detektion des Vorhandenseins von Skatol in einer Probe eines Schweinefettgewebes, **dadurch gekennzeichnet, dass** es mindestens die Schritte umfasst, die bestehen aus:

a) Unterziehen eines organischen Extrakts der Fettgewebeprobe einer Elektrochemilumineszenzreaktion;
b) Messen der Lumineszenzintensität während dem Schritt a) und, wenn die gemessene Lumineszenzintensität einen vorbestimmten Schwellenwert überschreitet, Folgern des Vorhandenseins von Skatol in der Fettgewebeprobe.

**2.** Verfahren nach Anspruch 1, wobei der organische Extrakt umfasst:

(1) ein aprotisches organisches Lösungsmittel, weniger als 1 Gew.-% an Wasser und (3) ein geschmolzenes Salz.

**3.** Verfahren nach Anspruch 2, das vor Schritt a) eine Zubereitung des organischen Extrakts umfasst, wobei die Zubereitung die Schritte umfasst, die bestehen aus:

i) Trennen des Fettes, das in der Fettgewebeprobe vorhanden ist, von den nicht fetthaltigen Elementen, die in der Fettgewebeprobe auch vorhanden sind;
ii) Dehydrieren des Fettes, das in der Fettgewebeprobe vorhanden ist;
iii) Auflösen des Fettes, das nach Schritt ii) erhalten wurde, in einem aprotischen organischen Lösungsmittel, um eine organische Lösung zu erhalten, und Erhitzen der organischen Lösung;
iv) Entfetten der organischen Lösung, die nach Schritt iii) erhalten wurde; und
v) Zusetzen eines trockenen geschmolzenen Salzes zur organischen Lösung, die nach Schritt iv) erhalten wurde;

wobei die Schritte i) und ii) nacheinander oder gleichzeitig durchgeführt werden können.

**4.** Verfahren nach Anspruch 3, wobei die Schritte i) und ii) gleichzeitig durchgeführt werden.

**5.** Verfahren nach Anspruch 4, wobei die Schritte i) und ii) ein Erhitzen der Fettgewebeprobe auf eine Temperatur von 100 °C bis 150 °C in einem offenen Behälter umfassen oder auf eine Temperatur unter 100 °C mit einem Vakuumzug.

**6.** Verfahren nach einem der Ansprüche 2 bis 5, wobei das organische Lösungsmittel Acetonitril, Dimethylsulfoxid, Propylencarbonat odery-Butyrolacton ist, vorzugsweise Acetonitril.

**7.** Verfahren nach einem der Ansprüche 2 bis 6, wobei das Erhitzen von Schritt iii) in einem geschlossenen Behälter bei einer Temperatur von 50 °C bis 80 °C für 10 Minuten bis 30 Minuten und unter Rühren durchgeführt wird.

**8.** Verfahren nach einem der Ansprüche 2 bis 7, wobei Schritt iv) eine Zentrifugation der nach Schritt iii) erhaltenen organischen Lösung umfasst, dann ein Erstarren des Fettanteils der organischen Lösung durch Halten der organischen Lösung bei einer Temperatur kleiner oder gleich 4 °C, jedoch höher als die Verfestigungstemperatur des aprotischen organischen Lösungsmittels, und eine Entfernung des Fettanteils, der erstarrt ist.

**9.** Verfahren nach einem der Ansprüche 2 bis 8, wobei das geschmolzene Salz Tetrafluoroborat, Hexafluorophosphat oder Tetrabutylammoniumperchlorat ist, wobei die Alkylgruppe 1 bis 4 Kohlenstoffatome umfasst, vorzugsweise Tetrabutylammoniumtetrafluorborat oder Tetrabutylammoniumhexafluorphosphat.

**10.** Verfahren nach einem der Ansprüche 2 bis 9, wobei der organische Extrakt 0,01 mol/L bis 1 mol/L geschmolzenes Salz umfasst, vorzugsweise 0,05 mol/L bis 0,5 mol/L, und noch besser 0,1 mol/L.

11. Verfahren nach einem der Ansprüche 2 bis 10, wobei der organische Extrakt zusätzlich eine starke Base umfasst.

12. Verfahren nach einem der Ansprüche 1 bis 11, wobei Schritt a) in einer elektrochemischen Zelle durchgeführt wird, die mindestens eine Arbeits- und eine Gegenelektrode umfasst, und wobei die Elektrochemilumineszenzreaktion das Anlegen an die Arbeitselektrode eines Kathodenpotentials gefolgt von einem Anodenpotential umfasst.

13. Verfahren nach Anspruch 12, wobei das Anlegen an die Arbeitselektrode eines Kathodenpotentials gefolgt von einem Anodenpotential durch Potentialabtastung, Potentialsprung oder durch eine Reihe von abwechselnd kathodischen und anodischen Potentialimpulsen durchgeführt wird.

14. Verfahren nach einem der Ansprüche 1 bis 13, wobei, nachdem das Vorhandensein von Skatol in Schritt b) gefolgert wurde, zusätzlich eine Quantifizierung des Skatols durchgeführt wird, das im organischen Extrakt vorhanden ist, durch Vergleich der in Schritt b) gemessenen maximalen Lumineszenzintensität mit einer Kalibrierungskurve, wobei die Quantifizierung in Schritt b) oder nach Schritt b) durchgeführt wird.

15. Verfahren zum Sortieren von ganzen männlichen Schweineleichen, **dadurch gekennzeichnet, dass** es die Durchführung eines Verfahrens nach einem der Ansprüche 1 bis 14 umfasst.

## Claims

1. Method for detecting the presence of skatole in a sample of a pig adipose tissue, **characterised in that** it comprises at least the steps of:

   a) subjecting an organic extract of the sample of adipose tissue to an electrochemiluminescence reaction;
   b) measuring the intensity of the luminescence during step a) and, if the measured luminescence intensity exceeds a predetermined threshold value, deducing the presence of skatole in the sample of adipose tissue.

2. Method according to claim 1, wherein the organic extract comprises: (1) an aprotic organic solvent, less than 1 wt.% water and (3) a ground salt.

3. Method according to claim 2, which comprises, prior to step a), a preparation of the organic extract, said preparation comprising the steps of:

   i) separating the fat present in the sample of adipose tissue from the non-fatty elements also present in the sample of adipose tissue;
   ii) dehydrating the fat present in the sample of adipose tissue;
   iii) dissolving the fat obtained at the end of step ii) in an aprotic organic solvent to obtain an organic solution and heating the organic solution;
   iv) degreasing the organic solution obtained at the end of step iii); and
   v) adding an anhydrous ground salt to the organic solution obtained at the end of step iv);

   and wherein steps i) and ii) may be carried out successively or simultaneously.

4. Method according to claim 3, wherein steps i) and ii) are carried out simultaneously.

5. Method according to claim 4, wherein steps i) and ii) comprise heating the sample of adipose tissue to a temperature of 100°C to 150°C in an open container, or to a temperature lower than 100°C with a vacuum drawing.

6. Method according to any of claims 2 to 5, wherein the organic solvent is acetonitrile, dimethylsulfoxide, propylene carbonate or $\gamma$-butyrolactone, preferably acetonitrile.

7. Method according to any of claims 2 to 6, wherein the heating of step iii) is carried out in a closed container, at a temperature of 50°C to 80°C, for 10 minutes to 30 minutes and with stirring.

8. Method according to any of claims 2 to 7, wherein step iv) comprises centrifugating the organic solution obtained at the end of step iii), then fixing the fatty part of the organic solution by maintaining the organic solution at a temperature lower than or equal to 4 °C but higher than the solidification temperature of the aprotic organic solvent,

and removing the fatty part that has been fixed.

**9.** Method according to any of claims 2 to 8, wherein the ground salt is a tetraalkylammonium tetrafluoroborate, hexafluorophosphate or perchlorate wherein the alkyl group comprises 1 to 4 carbon atoms, preferably tetrabutylammonium tetrafluoroborate or tetrabutylammonium hexafluorophosphate.

**10.** Method according to any of claims 2 to 9, wherein the organic extract comprises 0.01 mol/L to 1 mol/L, preferably 0.05 mol/L to 0.5 mol/L and, more preferably 0.1 mol/L of the ground salt.

**11.** Method according to any of claims 2 to 10, wherein the organic extract further comprises a strong base.

**12.** Method according to any of claims 1 to 11, wherein step a) is carried out in an electrochemical cell comprising at least one working electrode and one counter-electrode, and the electrochemiluminescence reaction comprises applying a cathodic potential followed by an anodic potential to the working electrode.

**13.** Method according to claim 12, wherein the application of a cathodic potential followed by an anodic potential to the working electrode is carried out by potential sweep, potential step or by a series of alternate cathodic and anodic potential pulses.

**14.** Method according to any of claims 1 to 13, wherein, if the presence of skatole has been deduced in step b), a quantification of skatole present in the organic extract is further carried out by comparing the maximum luminescence intensity measured during step b) with a calibration curve, the quantification being carried out during step b) or after step b).

**15.** Method for sorting carcasses of whole male pigs, **characterised in that** it comprises the implementation of a method according to any of claims 1 to 14.

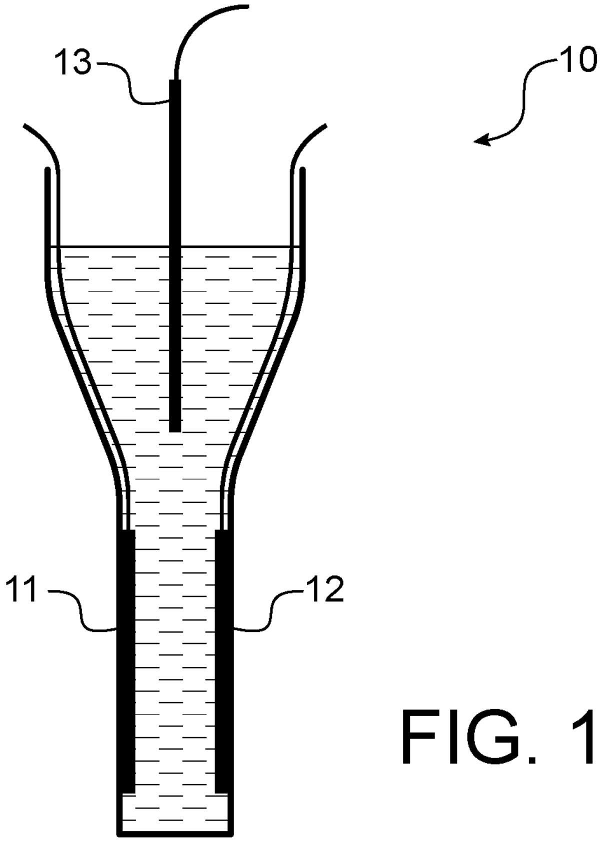


FIG. 1

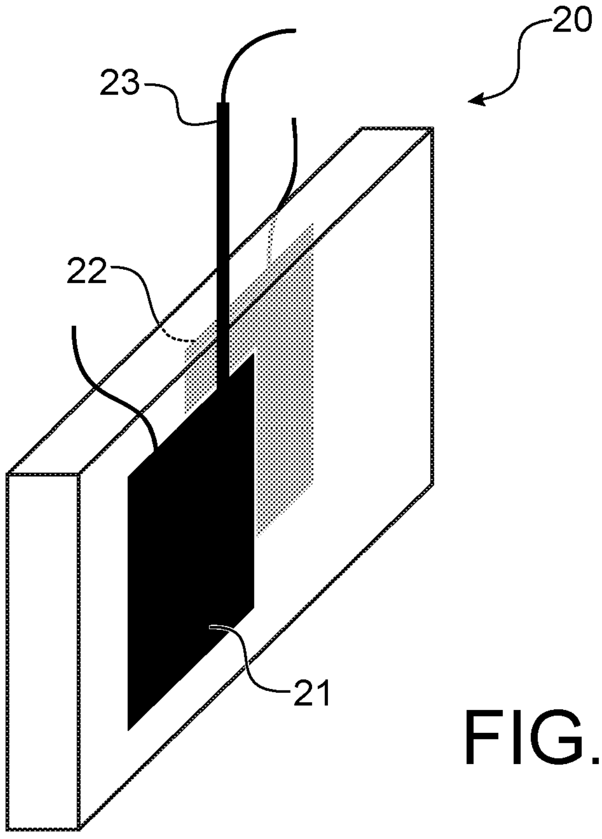


FIG. 2

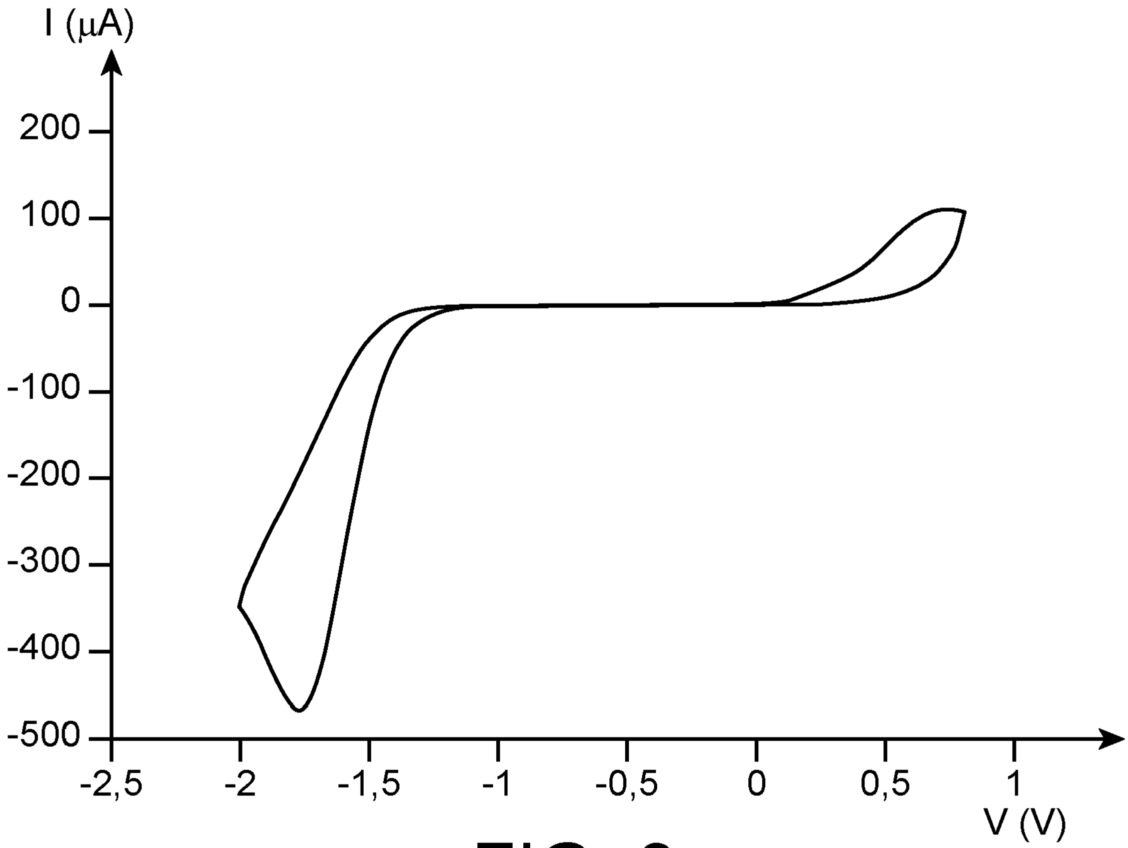
I (µA)
200
100
0
-100
-200
-300
-400
-500
-2,5
-2
-1,5
-1
-0,5
0
0,5
1
V (V)

FIG. 3

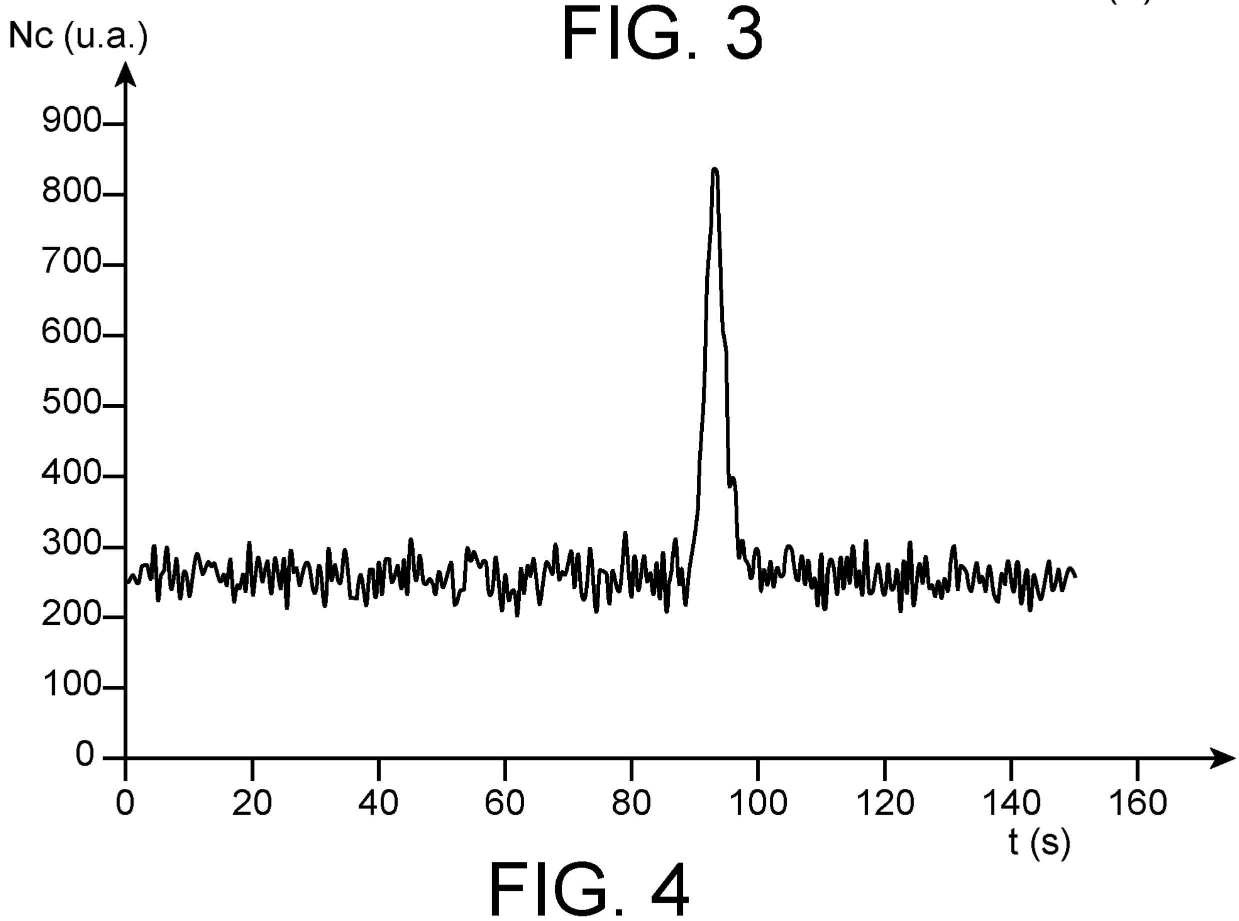
Nc (u.a.)
900
800
700
600
500
400
300
200
100
0
0
20
40
60
80
100
120
140
160
t (s)

FIG. 4

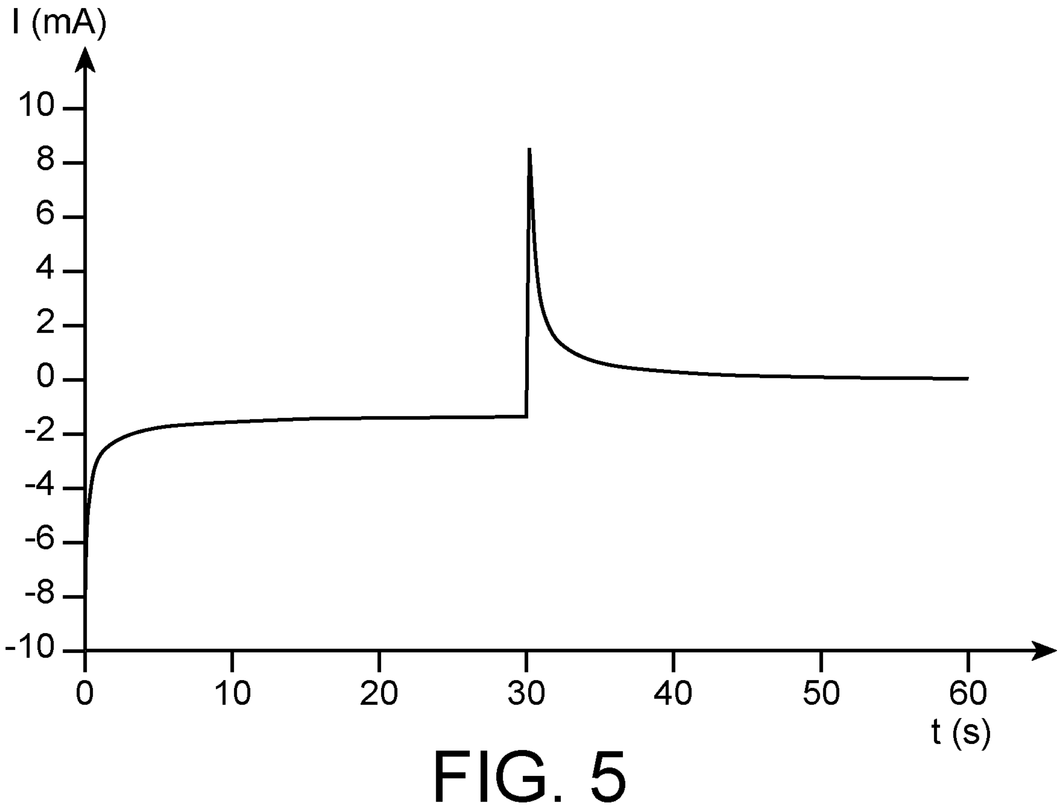
I (mA)
10
8
6
4
2
0
-2
-4
-6
-8
-10
0
10
20
30
40
50
60
t (s)

FIG. 5

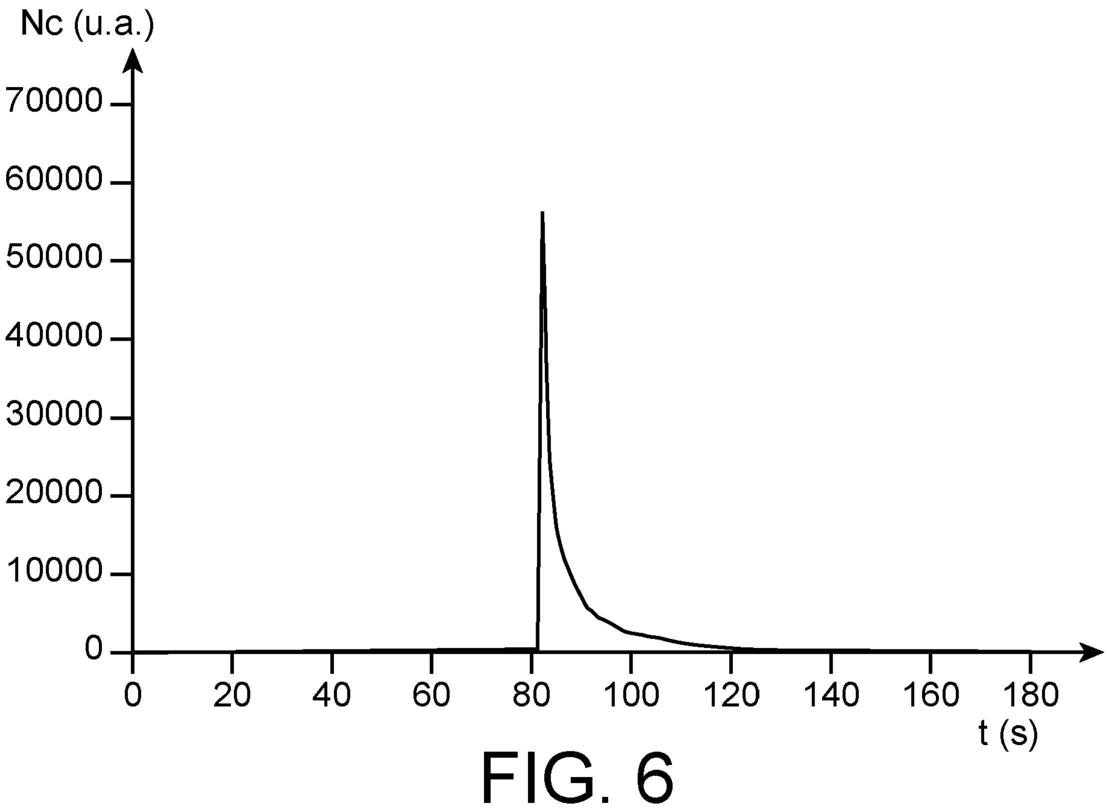
Nc (u.a.)
70000
60000
50000
40000
30000
20000
10000
0
0
20
40
60
80
100
120
140
160
180
t (s)

FIG. 6

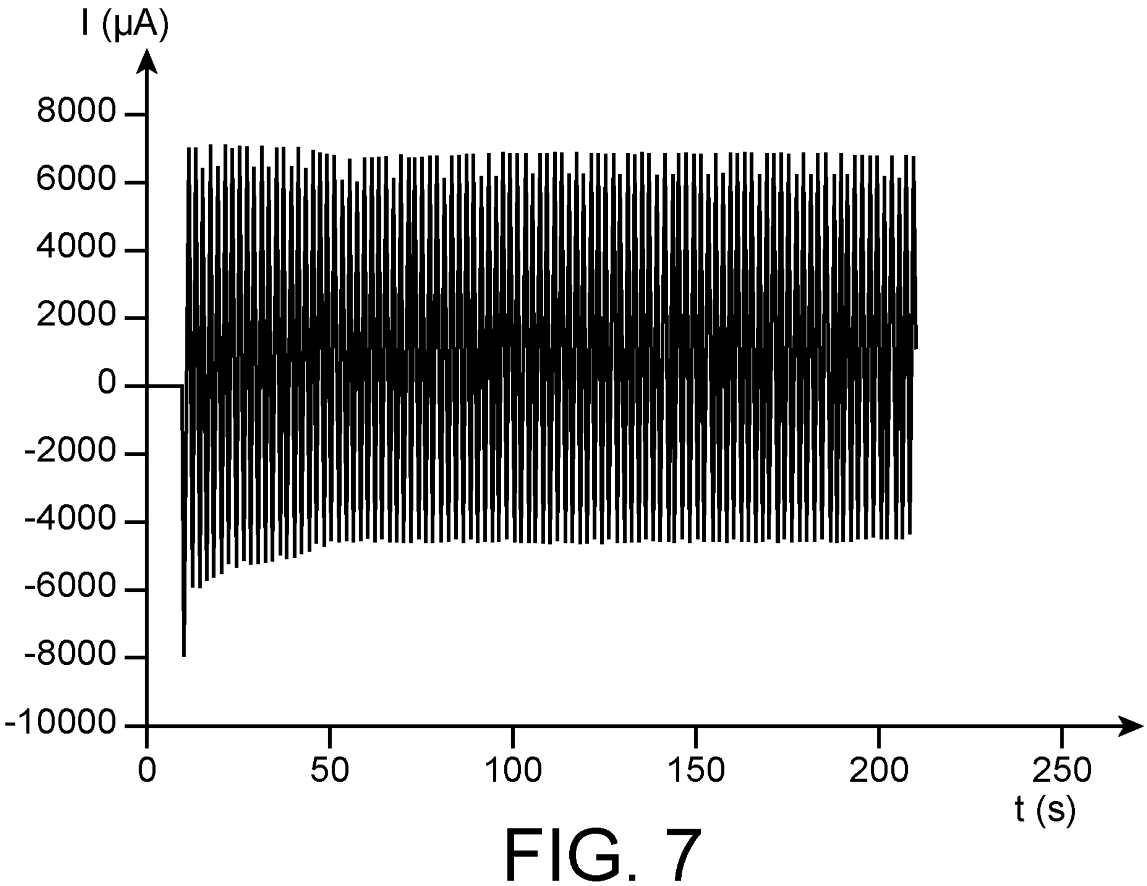
I (μA)
8000
6000
4000
2000
0
-2000
-4000
-6000
-8000
-10000
0
50
100
150
200
250
t (s)

FIG. 7

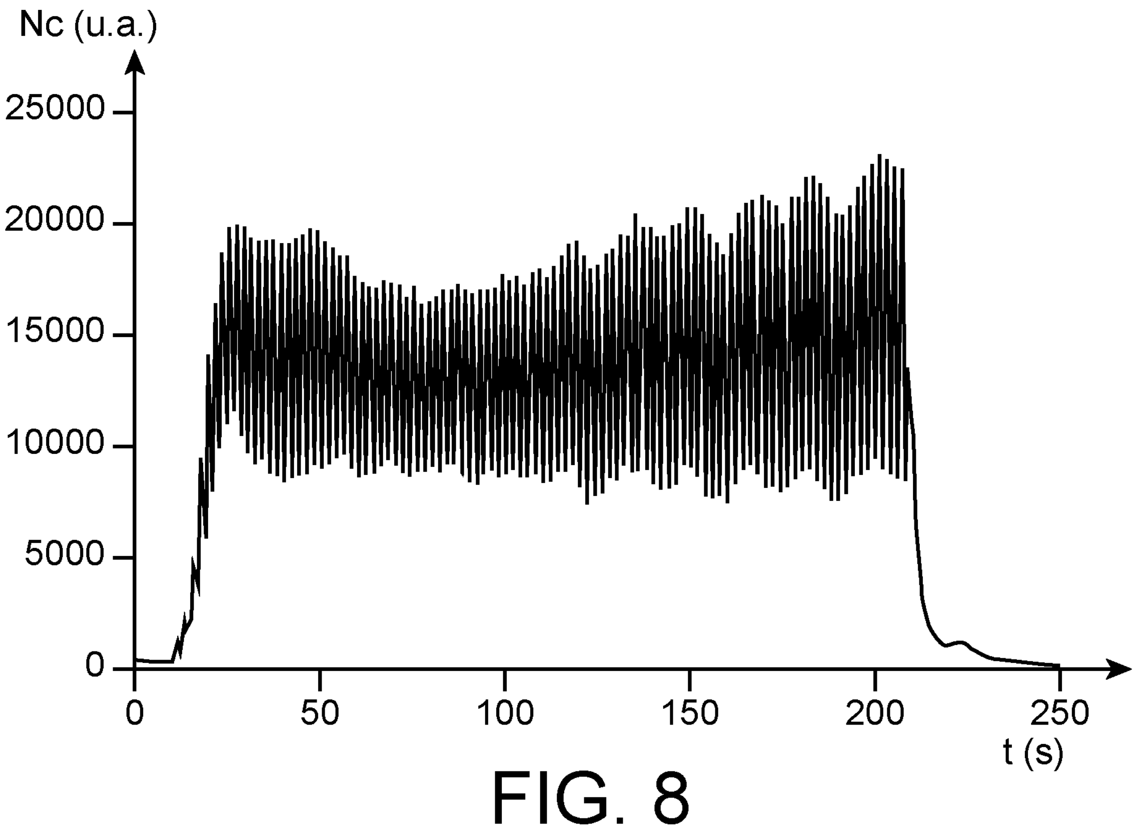
Nc (u.a.)
25000
20000
15000
10000
5000
0
0
50
100
150
200
250
t (s)

FIG. 8

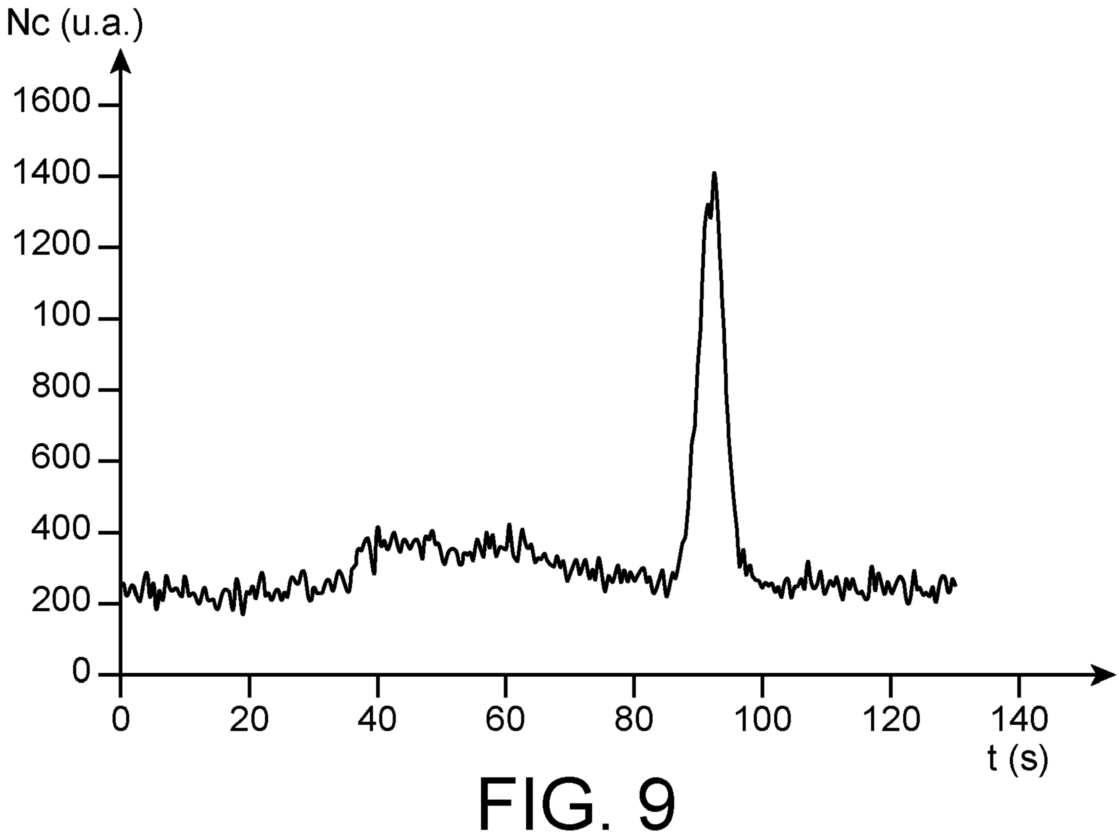
Nc (u.a.)
1600
1400
1200
100
800
600
400
200
0
0
20
40
60
80
100
120
140
t (s)

FIG. 9

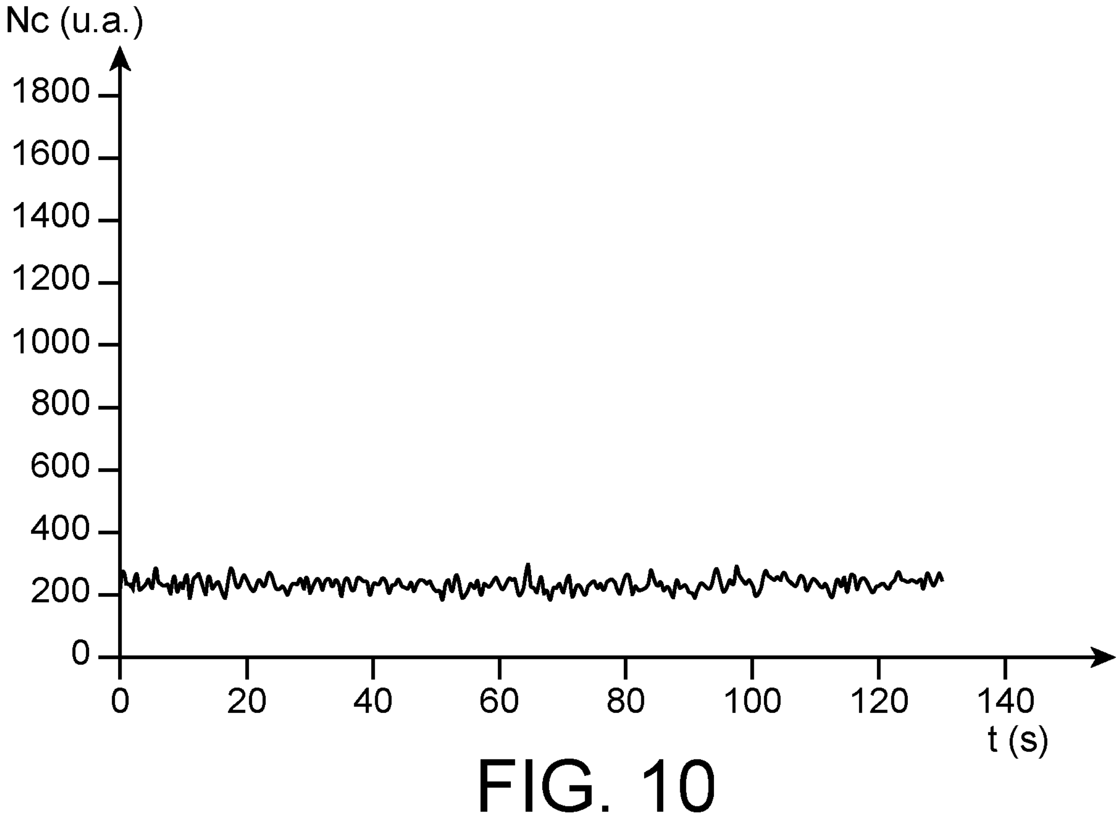
Nc (u.a.)
1800
1600
1400
1200
1000
800
600
400
200
0
0
20
40
60
80
100
120
140
t (s)

FIG. 10

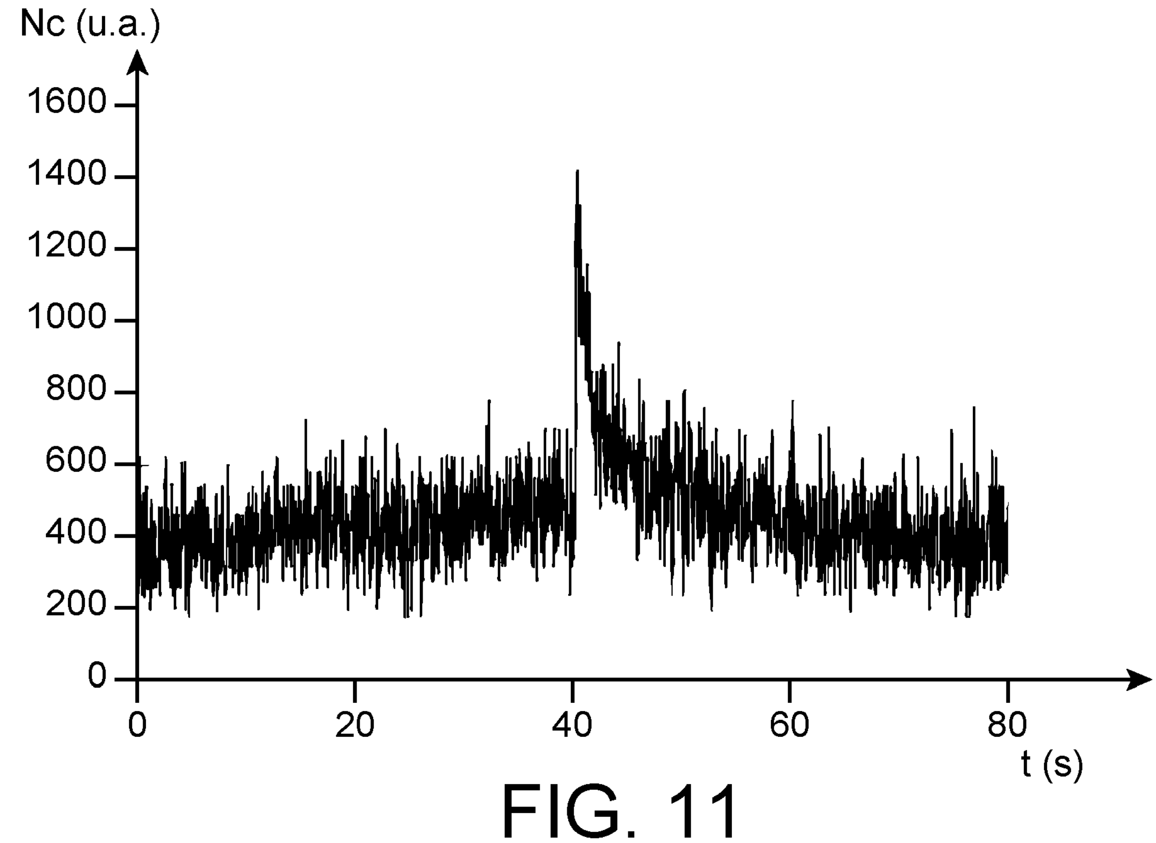
Nc (u.a.)
1600
1400
1200
1000
800
600
400
200
0
0
20
40
60
80
t (s)

FIG. 11

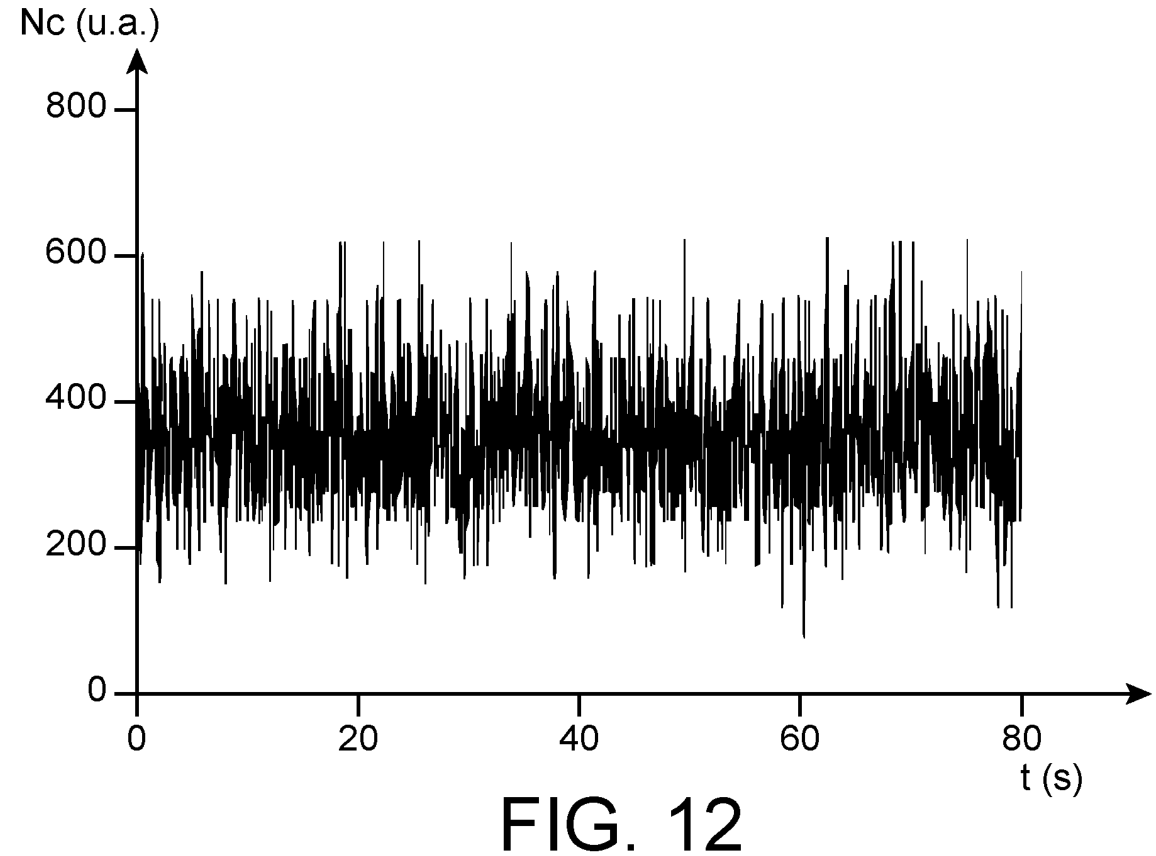
Nc (u.a.)
800
600
400
200
0
0
20
40
60
80
t (s)

FIG. 12

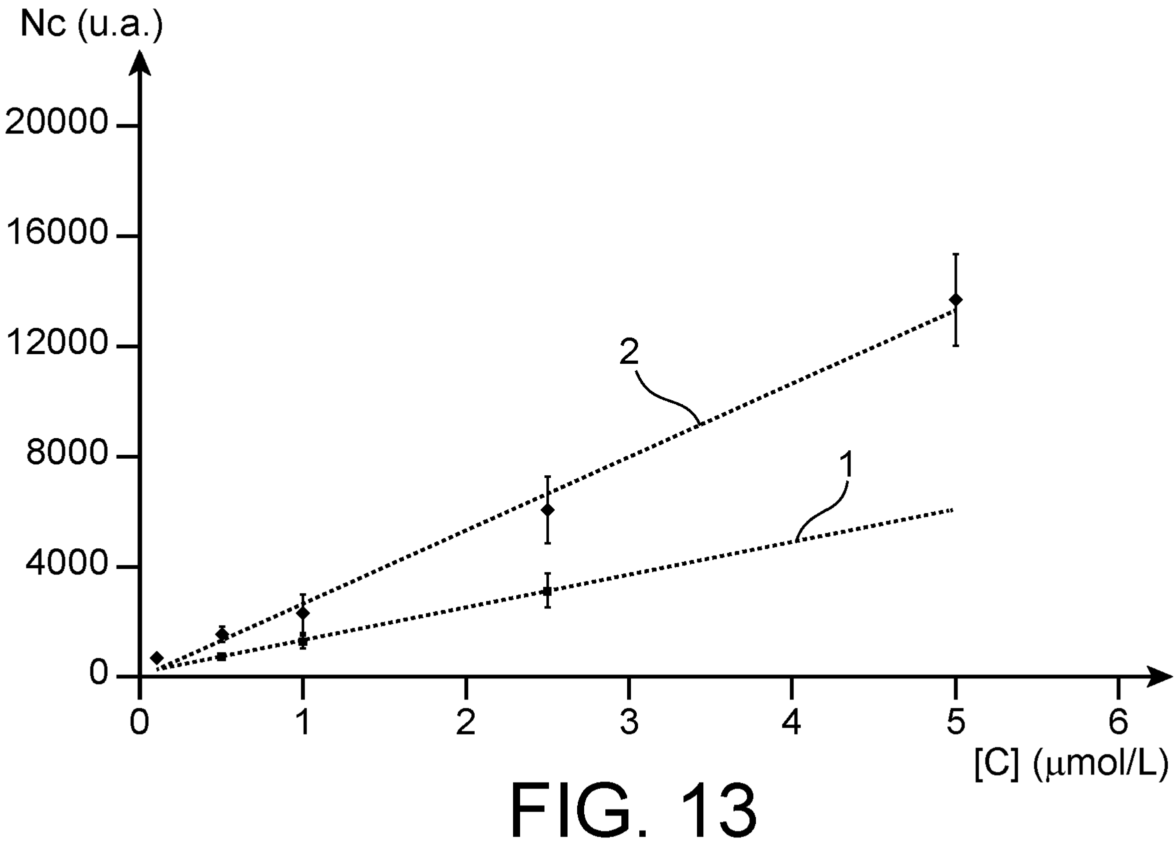


FIG. 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 8300928 A
- EP 2966441 A
- US 9121107 B
- US 9121107 A


### Littérature non-brevet citée dans la description


- **K. VERPLANKEN et al.** *Journal of Chromatography A,* 2016, vol. 1462, 124-133
- **M. P. AGUILAR-CABALLOS et al.** *Analytica Chimica Acta,* 2002, vol. 460, 271-277
- **X. LIU et al.** *Meat Science,* 2016, vol. 116, 133-139
- **J. S. VESTERGAARD et al.** *Meat Science,* 2006, vol. 74, 564-577
- **T. OKAJIMA ; T. OHSAKA.** *Journal of Electroanalytical Chemistry,* 2002, vol. 523, 34-39
- *CHEMICAL ABSTRACTS,* 7646-69-7